# EUROPEAN PATENT APPLICATION

(11) **EP 3 246 434 A1**
(43) Date of publication of application: **22.11.2017**
(21) Application number: 16737531.0
(22) Date of filing: 13.01.2016
(51) Int. Cl.: C40B 50/02, C40B 40/10, C07K 16/00

(54) **METHOD FOR PREPARING NOVEL ANTIBODY LIBRARY AND LIBRARY PREPARED THEREBY**

(30) Priority: 13.01.2015 KR 20150006358
(71) Applicant: Ewha University-Industry Collaboration Foundation, Seoul 03760 (KR); Medicinal Bioconvergence Research Center, Suwon-si, Gyeonggi-do 16229 (KR)
(72) Inventor: SHIM, Hyun Bo, Seoul 06005 (KR); KIM, Ji Hye, Incheon 22782 (KR); BAI, Xuelian, Seoul 04106 (KR)
(74) Representative: Docherty, Robert Charles
(86) International application number: PCT/KR2016/000320
(87) International publication number: WO 2016/114567

(57) **Abstract**

The present invention relates to a method for preparing a novel antibody library and a library prepared thereby. The antibody library prepared according to the present invention contains antibodies having excellent physical properties against a plurality of antigens, thereby having functional diversity and containing a plurality of unique sequences, and thus can be favorably used as an antibody library.

<110>     Ewha University - Industry Collaboration Foundation

<120>     Novel method for generating a antibody library and the generated
          library therefrom

<130>     OPA15317

<150>     KR 10-2015-0006358
<151>     2015-01-13

<160>     252

<170>     KoPatentIn 3.0

<210>     1
<211>     19
<212>     DNA
<213>     Artificial Sequence

<220>
<223>     OPALS-h1-f


<400>     1
cagcggattc accttcagc                                                    19


<210>     2
<211>     18
<212>     DNA
<213>     Artificial Sequence

<220>
<223>     OPALS-h1-b primer


<400>     2
aggtgcttgg cgaaccca                                                     18


<210>     3
<211>     18
<212>     DNA
<213>     Artificial Sequence

<220>
<223>     OPALS-h2-f primer


<400>     3
ggcctggaat gggtgagc                                                     18


<210>     4
<211>     18
<212>     DNA
<213>     Artificial Sequence

<220>
<223>     OPALS-h2-b primer


<400>     4

gcggctgatg gtaaagcg                                                    18

<210>    5
<211>    20
<212>    DNA
<213>    Artificial Sequence

<220>
<223>    OPALS-h3-f primer

<400>    5
ggacaccgca gtctactact                                                  20

<210>    6
<211>    19
<212>    DNA
<213>    Artificial Sequence

<220>
<223>    OPALS-h3-b primer

<400>    6
caccagagta ccttgtccc                                                   19

<210>    7
<211>    18
<212>    DNA
<213>    Artificial Sequence

<220>
<223>    OPALS-k1-f primer

<400>    7
cgcgcaacac tgtcatgc                                                    18

<210>    8
<211>    19
<212>    DNA
<213>    Artificial Sequence

<220>
<223>    OPALS-k1-b primer

<400>    8
tggagcctga cctggtttc                                                   19

<210>    9
<211>    18
<212>    DNA
<213>    Artificial Sequence

<220>
<223>    OPALS-k2-f primer

<400>    9
ccaggtcagg ctccacgt                                                    18


<210>    10
<211>    19
<212>    DNA
<213>    Artificial Sequence

<220>
<223>    OPALS-k2-b primer


<400>    10
accgcttcca gatcctgag                                                   19


<210>    11
<211>    19
<212>    DNA
<213>    Artificial Sequence

<220>
<223>    OPALS-k3-f primer


<400>    11
ctggaacctg aggactttg                                                   19


<210>    12
<211>    18
<212>    DNA
<213>    Artificial Sequence

<220>
<223>    OPALS-k3-b primer


<400>    12
actttagtgc cctgaccg                                                    18


<210>    13
<211>    19
<212>    DNA
<213>    Artificial Sequence

<220>
<223>    OPALS-l1-f primer


<400>    13
gcgcgtgact attagctgt                                                   19


<210>    14
<211>    19
<212>    DNA
<213>    Artificial Sequence

<220>

<223>    OPALS-11-b primer

<400>    14
aggtgcagtt ccaggcagt                                                    19

<210>    15
<211>    19
<212>    DNA
<213>    Artificial Sequence

<220>
<223>    OPALS-12-f primer

<400>    15
gcctggaact gcacctaag                                                    19

<210>    16
<211>    19
<212>    DNA
<213>    Artificial Sequence

<220>
<223>    OPALS-12-b primer

<400>    16
gcctgatttg ctaccgcta                                                    19

<210>    17
<211>    19
<212>    DNA
<213>    Artificial Sequence

<220>
<223>    OPALS-13-f primer

<400>    17
cttcgctccg aagatgaag                                                    19

<210>    18
<211>    18
<212>    DNA
<213>    Artificial Sequence

<220>
<223>    OPALS-13-b primer

<400>    18
gtcagcttgg taccgcca                                                     18

<210>    19
<211>    717
<212>    DNA
<213>    Artificial Sequence

<220>
<223>    scFv VH3-23_linker_VK3-A27


<400>    19
gaagtgcagc tgctggaaag tggaggtgga ctggtgcagc ctggcggcag cctgcgcctg        60

agctgtgccg ccagcggatt caccttcagc agctatgcca tgagctgggt tcgccaagca       120

cctggcaaag gcctggaatg ggtgagcgcc atcagcggca gcggcggcag cacctattat       180

gccgatagcg tgaaaggccg ctttaccatc agccgcgata acagcaaaaa caccctgtat       240

ctgcagatga acagcctgcg cgccgaggac accgcagtct actactgtgc caaaaacaaa       300

ctgtggtttg attattgggg acaaggtact ctggtgaccg tgagcagcgg tggaggaggt       360

agcggaggtg gtggatctgg aggtggaggt agtgaaatcg tgctgaccca gagccctggc       420

accctgagcc tgagccctgg cgaacgcgca acactgtcat gccgcgccag ccagagcgtg       480

agcagcagct atctggcctg gtatcagcag aaaccaggtc aggctccacg tctgctgatc       540

tatggcgcca gcagccgcgc caccggcatc cctgatcgct tctcaggatc tggaagcggt       600

accgatttta ccctgaccat cagccgcctg gaacctgagg actttgccgt gtattattgt       660

cagcagtatg gcagcagccc ttggaccttc ggtcagggca ctaaagtgga aatcaaa          717


<210>    20
<211>    723
<212>    DNA
<213>    Artificial Sequence

<220>
<223>    scFv VH3-23_linker_VL1g


<400>    20
gaagtgcagc tgctggaaag tggaggtgga ctggtgcagc ctggcggcag cctgcgcctg        60

agctgtgccg ccagcggatt caccttcagc agctatgcca tgagctgggt tcgccaagca       120

cctggcaaag gcctggaatg ggtgagcgcc atcagcggca gcggcggcag cacctattat       180

gccgatagcg tgaaaggccg ctttaccatc agccgcgata acagcaaaaa caccctgtat       240

ctgcagatga acagcctgcg cgccgaggac accgcagtct actactgtgc caaaaacaaa       300

ctgtggtttg attattgggg acaaggtact ctggtgaccg tgagcagcgg tggaggaggt       360

agcggaggtg gtggatctgg aggtggaggt agtcagagcg tgctgaccca gcctcctagc       420

gcctccggta caccaggaca gcgcgtgact attagctgta gcggcagcag cagcaacatc       480

ggcagcaact atgtgtattg gtaccagcaa ctgcctggaa ctgcacctaa gctgctgatc       540

tatcgcaaca accagcgccc tagcggcgtg cctgatcgct ttagcggtag caaatcaggc       600

accagcgcca gcctggccat cagcggcctt cgctccgaag atgaagccga ttattattgt       660

gccgcctggg atgatagcct gagcggctat gtgtttggtg gcggtaccaa gctgaccgtg 720

ctg 723

<210> 21
<211> 18
<212> DNA
<213> Artificial Sequence

<220>
<223> OPALS-H1-rc-f primer

<400> 21
tgggttcgcc aagcacct 18

<210> 22
<211> 18
<212> DNA
<213> Artificial Sequence

<220>
<223> OPALS-H2-rc-f primer

<400> 22
cgctttacca tcagccgc 18

<210> 23
<211> 19
<212> DNA
<213> Artificial Sequence

<220>
<223> OPALS-H3-rc-f primer

<400> 23
gggacaaggt actctggtg 19

<210> 24
<211> 19
<212> DNA
<213> Artificial Sequence

<220>
<223> OPALS-L1-rc-f primer

<400> 24
actgcctgga actgcacct 19

<210> 25
<211> 19
<212> DNA
<213> Artificial Sequence

<220>

<223>    OPALS-L2-rc-f primer

<400>    25
tagcggtagc aaatcaggc                                                    19

<210>    26
<211>    18
<212>    DNA
<213>    Artificial Sequence

<220>
<223>    OPALS-L3-rc-f primer

<400>    26
tggcggtacc aagctgac                                                     18

<210>    27
<211>    19
<212>    DNA
<213>    Artificial Sequence

<220>
<223>    OPALS-K1-rc-f primer

<400>    27
gaaaccaggt caggctcca                                                    19

<210>    28
<211>    19
<212>    DNA
<213>    Artificial Sequence

<220>
<223>    OPALS-K2-rc-f primer

<400>    28
ctcaggatct ggaagcggt                                                    19

<210>    29
<211>    18
<212>    DNA
<213>    Artificial Sequence

<220>
<223>    OPALS-K3-rc-f primer

<400>    29
cggtcagggc actaaagt                                                     18

<210>    30
<211>    19
<212>    DNA
<213>    Artificial Sequence

<220>
<223>    OPALS-H1-rc-b primer


<400>    30
gctgaaggtg aatccgctg                                                    19


<210>    31
<211>    18
<212>    DNA
<213>    Artificial Sequence

<220>
<223>    OPALS-H2-rc-b primer


<400>    31
gctcacccat tccaggcc                                                     18


<210>    32
<211>    20
<212>    DNA
<213>    Artificial Sequence

<220>
<223>    OPALS-H3-rc-b primer


<400>    32
agtagtagac tgcggtgtcc                                                   20


<210>    33
<211>    19
<212>    DNA
<213>    Artificial Sequence

<220>
<223>    OPALS-L1-rc-b primer


<400>    33
acagctaata gtcacgcgc                                                    19


<210>    34
<211>    19
<212>    DNA
<213>    Artificial Sequence

<220>
<223>    OPALS-L2-rc-b primer


<400>    34
cttaggtgca gttccaggc                                                    19


<210>    35
<211>    19

<212>    DNA
<213>    Artificial Sequence

<220>
<223>    OPALS-L3-rc-b primer


<400>    35
cttcatcttc ggagcgaag                                                    19


<210>    36
<211>    18
<212>    DNA
<213>    Artificial Sequence

<220>
<223>    OPALS-K1-rc-b primer


<400>    36
gcatgacagt gttgcgcg                                                     18


<210>    37
<211>    18
<212>    DNA
<213>    Artificial Sequence

<220>
<223>    OPALS-K2-rc-b primer


<400>    37
acgtggagcc tgacctgg                                                     18


<210>    38
<211>    19
<212>    DNA
<213>    Artificial Sequence

<220>
<223>    OPALS-K3-rc-b primer


<400>    38
caaagtcctc aggttccag                                                    19


<210>    39
<211>    20
<212>    DNA
<213>    Artificial Sequence

<220>
<223>    pC3x-b primer


<400>    39
aaccatcgat agcagcaccg                                                   20

<210>    40
<211>    18
<212>    DNA
<213>    Artificial Sequence

<220>
<223>    pUC57-b primer


<400>    40
ttcgccattc aggctgcg                                                    18


<210>    41
<211>    19
<212>    DNA
<213>    Artificial Sequence

<220>
<223>    pC3x-f primer


<400>    41
gcacgacagg tttcccgac                                                   19


<210>    42
<211>    19
<212>    DNA
<213>    Artificial Sequence

<220>
<223>    lFR3-f primer


<400>    42
ctggccatca gcggccttc                                                   19


<210>    43
<211>    18
<212>    DNA
<213>    Artificial Sequence

<220>
<223>    lFR3-b primer


<400>    43
ctgggtcagc acgatttc                                                    18


<210>    44
<211>    18
<212>    DNA
<213>    Artificial Sequence

<220>
<223>    kFR1-f primer


<400>    44
gaaatcgtgc tgacccag                                                    18

<210>    45
<211>    18
<212>    DNA
<213>    Artificial Sequence

<220>
<223>    kFR1-b primer


<400>    45
ctgggtcagc acgatttc                                                    18


<210>    46
<211>    18
<212>    DNA
<213>    Artificial Sequence

<220>
<223>    OPALS-FR4-f primer


<400>    46
ctggtgaccg tgagcagc                                                    18


<210>    47
<211>    18
<212>    DNA
<213>    Artificial Sequence

<220>
<223>    OPALS-FR4-b primer


<400>    47
gctgctcacg gtcaccag                                                    18


<210>    48
<211>    19
<212>    DNA
<213>    Artificial Sequence

<220>
<223>    pC3-seq primer


<400>    48
gtgagcggat aacaattga                                                   19


<210>    49
<211>    19
<212>    DNA
<213>    Artificial Sequence

<220>
<223>    dp-seq primer

<400>    49
agaagcgtag tccggaacg                                                    19


<210>    50
<211>    627
<212>    DNA
<213>    Artificial Sequence

<220>
<223>    (VH3-23)-linker-(Vk3-A27) frame work


<400>    50
gaagtgcagc tgctggaaag tggaggtgga ctggtgcagc ctggcggcag cctgcgcctg      60

agctgtgccg ccagcggatt caccttcagc nnnnnnnnnn nnnnntgggt tcgccaagca     120

cctggcaaag gcctggaatg ggtgnnnnnn nnnnnnnnnc gctttaccat cagccgcgat     180

aacagcaaaa acaccctgta tctgcagatg aacagcctgc gcgccgagga caccgcagtc     240

tactactgtn nnnnnnnnnn nnnntgggga caaggtactc tggtgaccgt gagcagcggt     300

ggaggaggta gcggaggtgg tggatctgga ggtggaggta gtgaaatcgt gctgacccag     360

agccctggca ccctgagcct gagccctggc gaacgcgcaa cactgtcatg cnnnnnnnnn     420

nnnnnntggt atcagcagaa accaggtcag gctccacgtc tgctgatcta tnnnnnnnnn     480

nnnnnnggca tccctgatcg cttctcagga tctggaagcg gtaccgattt taccctgacc     540

atcagccgcc tggaacctga ggactttgcc gtgtattatt gtnnnnnnnn nnnnnnnttc     600

ggtcagggca ctaaagtgga aatcaaa                                         627


<210>    51
<211>    211
<212>    PRT
<213>    Artificial Sequence

<220>
<223>    (VH3-23)-linker-(Vk3-A27) frame work a.a.


<400>    51
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Xaa Xaa
            20                  25                  30

Xaa Xaa Xaa Trp Val Arg Gln Ala Pro Gly Lys Gly Leu Glu Trp Val
        35                  40                  45

Xaa Xaa Xaa Xaa Xaa Arg Phe Thr Ile Ser Arg Asp Asn Ser Lys Asn
    50                  55                  60

Thr Leu Tyr Leu Gln Met Asn Ser Leu Arg Ala Glu Asp Thr Ala Val
65                  70                  75                  80

Tyr Tyr Cys Ala Lys Xaa Xaa Xaa Xaa Xaa Trp Gly Gln Gly Thr Leu

85                  90                  95

Val Thr Val Ser Ser Gly Gly Gly Gly Ser Gly Gly Gly Gly Ser Gly
            100                 105                 110

Gly Gly Gly Ser Glu Ile Val Leu Thr Gln Ser Pro Gly Thr Leu Ser
        115                 120                 125

Leu Ser Pro Gly Glu Arg Ala Thr Leu Ser Cys Xaa Xaa Xaa Xaa Xaa
    130                 135                 140

Trp Tyr Gln Gln Lys Pro Gly Gln Ala Pro Arg Leu Leu Ile Tyr Xaa
145                 150                 155                 160

Xaa Xaa Xaa Xaa Gly Ile Pro Asp Arg Phe Ser Gly Ser Gly Ser Gly
                165                 170                 175

Thr Asp Phe Thr Leu Thr Ile Ser Arg Leu Glu Pro Glu Asp Phe Ala
            180                 185                 190

Val Tyr Tyr Cys Xaa Xaa Xaa Xaa Xaa Phe Gly Gln Gly Thr Lys Val
        195                 200                 205

Glu Ile Lys
    210

<210>    52
<211>    624
<212>    DNA
<213>    Artificial Sequence

<220>
<223>    (VH3-23)-linker-(Vl1g) frame work

<400>    52
gaagtgcagc tgctggaaag tggaggtgga ctggtgcagc ctggcggcag cctgcgcctg      60

agctgtgccg ccagcggatt caccttcagc nnnnnnnnnn nnnnntgggt tcgccaagca     120

cctggcaaag gcctggaatg ggtgnnnnnn nnnnnnnnnc gctttaccat cagccgcgat     180

aacagcaaaa acaccctgta tctgcagatg aacagcctgc gcgccgagga caccgcagtc     240

tactactgtn nnnnnnnnnn nnnntgggga caaggtactc tggtgaccgt gagcagcggt     300

ggaggaggta gcggaggtgg tggatctgga ggtggaggta gtcagagcgt gctgacccag     360

cctcctagcg cctccggtac accaggacag cgcgtgacta ttagctgtnn nnnnnnnnnn     420

nnntggtacc agcaactgcc tggaactgca cctaagctgc tgatctatnn nnnnnnnnnn     480

nnnggcgtgc ctgatcgctt tagcggtagc aaatcaggca ccagcgccag cctggccatc     540

agcggccttc gctccgaaga tgaagccgat tattattgtn nnnnnnnnnn nnnntttggt     600

ggcggtacca agctgaccgt gctg                                            624

<210>    53
<211>    210
<212>    PRT

<213>    Artificial Sequence

<220>
<223>    (VH3-23)-linker-(Vl1g) frame work a.a.


<400>    53
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Xaa Xaa
            20                  25                  30

Xaa Xaa Xaa Trp Val Arg Gln Ala Pro Gly Lys Gly Leu Glu Trp Val
        35                  40                  45

Xaa Xaa Xaa Xaa Xaa Arg Phe Thr Ile Ser Arg Asp Asn Ser Lys Asn
    50                  55                  60

Thr Leu Tyr Leu Gln Met Asn Ser Leu Arg Ala Glu Asp Thr Ala Val
65                  70                  75                  80

Tyr Tyr Cys Ala Lys Xaa Xaa Xaa Xaa Xaa Trp Gly Gln Gly Thr Leu
                85                  90                  95

Val Thr Val Ser Ser Gly Gly Gly Gly Ser Gly Gly Gly Gly Ser Gly
            100                 105                 110

Gly Gly Gly Ser Gln Ser Val Leu Thr Gln Pro Pro Ser Ala Ser Gly
        115                 120                 125

Thr Pro Gly Gln Arg Val Thr Ile Ser Cys Xaa Xaa Xaa Xaa Xaa Trp
    130                 135                 140

Tyr Gln Gln Leu Pro Gly Thr Ala Pro Lys Leu Leu Ile Tyr Xaa Xaa
145                 150                 155                 160

Xaa Xaa Xaa Gly Val Pro Asp Arg Phe Ser Gly Ser Lys Ser Gly Thr
                165                 170                 175

Ser Ala Ser Leu Ala Ile Ser Gly Leu Arg Ser Glu Asp Glu Ala Asp
            180                 185                 190

Tyr Tyr Cys Xaa Xaa Xaa Xaa Xaa Phe Gly Gly Gly Thr Lys Leu Thr
        195                 200                 205

Val Leu
    210


<210>    54
<211>    5
<212>    PRT
<213>    Homo sapiens

<400>    54
Asp His Tyr Met Asp
1               5


<210>    55
<211>    5
<212>    PRT

<213>    Homo sapiens

<400>    55
Asp Tyr Ala Met His
1               5

<210>    56
<211>    5
<212>    PRT
<213>    Homo sapiens

<400>    56
Asp Tyr Ala Met Ser
1               5

<210>    57
<211>    5
<212>    PRT
<213>    Homo sapiens

<400>    57
Asp Tyr Gly Met Ser
1               5

<210>    58
<211>    5
<212>    PRT
<213>    Homo sapiens

<400>    58
Asp Tyr Thr Met His
1               5

<210>    59
<211>    5
<212>    PRT
<213>    Homo sapiens

<400>    59
Asp Tyr Tyr Met His
1               5

<210>    60
<211>    5
<212>    PRT
<213>    Homo sapiens

<400>    60
Asp Tyr Tyr Met Ser
1               5

<210>    61
<211>    5
<212>    PRT
<213>    Homo sapiens

<400>    61

Glu Leu Ser Met His
1               5

<210>   62
<211>   5
<212>   PRT
<213>   Homo sapiens

<400>   62
Gly Ser Ala Met His
1               5

<210>   63
<211>   5
<212>   PRT
<213>   Homo sapiens

<400>   63
Gly Tyr Tyr Met His
1               5

<210>   64
<211>   5
<212>   PRT
<213>   Homo sapiens

<400>   64
Gly Tyr Tyr Trp Ser
1               5

<210>   65
<211>   5
<212>   PRT
<213>   Homo sapiens

<400>   65
Asn Ala Trp Met Ser
1               5

<210>   66
<211>   5
<212>   PRT
<213>   Homo sapiens

<400>   66
Ser Asn Glu Met Ser
1               5

<210>   67
<211>   5
<212>   PRT
<213>   Homo sapiens

<400>   67
Ser Asn Tyr Met Ser
1               5

<210> 68
<211> 5
<212> PRT
<213> Homo sapiens

<400> 68
Ser Ser Ala Val Gln
1               5

<210> 69
<211> 5
<212> PRT
<213> Homo sapiens

<400> 69
Ser Tyr Ala Ile Ser
1               5

<210> 70
<211> 5
<212> PRT
<213> Homo sapiens

<400> 70
Ser Tyr Ala Met His
1               5

<210> 71
<211> 5
<212> PRT
<213> Homo sapiens

<400> 71
Ser Tyr Ala Met Asn
1               5

<210> 72
<211> 5
<212> PRT
<213> Homo sapiens

<400> 72
Ser Tyr Ala Met Ser
1               5

<210> 73
<211> 5
<212> PRT
<213> Homo sapiens

<400> 73
Ser Tyr Asp Ile Asn
1               5

<210> 74
<211> 5

<212>    PRT
<213>    Homo sapiens

<400>    74
Ser Tyr Asp Met His
1               5

<210>    75
<211>    5
<212>    PRT
<213>    Homo sapiens

<400>    75
Ser Tyr Gly Ile Ser
1               5

<210>    76
<211>    5
<212>    PRT
<213>    Homo sapiens

<400>    76
Ser Tyr Gly Met His
1               5

<210>    77
<211>    5
<212>    PRT
<213>    Homo sapiens

<400>    77
Ser Tyr Ser Met Asn
1               5

<210>    78
<211>    5
<212>    PRT
<213>    Homo sapiens

<400>    78
Ser Tyr Trp Ile Gly
1               5

<210>    79
<211>    5
<212>    PRT
<213>    Homo sapiens

<400>    79
Ser Tyr Trp Ile Ser
1               5

<210>    80
<211>    5
<212>    PRT
<213>    Homo sapiens

<400>    80
Ser Tyr Trp Met His
1               5

<210>    81
<211>    5
<212>    PRT
<213>    Homo sapiens

<400>    81
Ser Tyr Trp Met Ser
1               5

<210>    82
<211>    5
<212>    PRT
<213>    Homo sapiens

<400>    82
Ser Tyr Tyr Met His
1               5

<210>    83
<211>    5
<212>    PRT
<213>    Homo sapiens

<400>    83
Ser Tyr Tyr Trp Ser
1               5

<210>    84
<211>    5
<212>    PRT
<213>    Homo sapiens

<400>    84
Tyr Arg Tyr Leu His
1               5

<210>    85
<211>    16
<212>    PRT
<213>    Homo sapiens

<400>    85
Ala Ile Gly Thr Ala Gly Asp Thr Tyr Tyr Pro Gly Ser Val Lys Gly
1               5                   10                  15

<210>    86
<211>    16
<212>    PRT
<213>    Homo sapiens

<400>    86
Glu Ile Asn His Ser Gly Ser Thr Asn Tyr Asn Pro Ser Leu Lys Ser
1               5                   10                  15

<210>    87
<211>    16
<212>    PRT
<213>    Homo sapiens

<400>    87
Glu Ile Tyr His Ser Gly Ser Thr Asn Tyr Asn Pro Ser Leu Lys Ser
1               5                   10                  15

<210>    88
<211>    16
<212>    PRT
<213>    Homo sapiens

<400>    88
His Ile Phe Ser Asn Asp Glu Lys Ser Tyr Ser Thr Ser Leu Lys Ser
1               5                   10                  15

<210>    89
<211>    16
<212>    PRT
<213>    Homo sapiens

<400>    89
Leu Ile Tyr Trp Asn Asp Asp Lys Arg Tyr Ser Pro Ser Leu Lys Ser
1               5                   10                  15

<210>    90
<211>    16
<212>    PRT
<213>    Homo sapiens

<400>    90
Arg Ile Asp Trp Asp Asp Asp Lys Phe Tyr Ser Thr Ser Leu Lys Thr
1               5                   10                  15

<210>    91
<211>    16
<212>    PRT
<213>    Homo sapiens

<400>    91
Ser Ile Tyr His Ser Gly Ser Thr Tyr Tyr Asn Pro Ser Leu Lys Ser
1               5                   10                  15

<210>    92
<211>    16
<212>    PRT
<213>    Homo sapiens

<400>    92
Ser Ile Tyr Tyr Ser Gly Ser Thr Tyr Tyr Asn Pro Ser Leu Lys Ser
1               5                   10                  15

<210>    93

<211>    16
<212>    PRT
<213>    Homo sapiens

<400>    93
Val Ile Tyr Ser Gly Gly Ser Thr Tyr Tyr Ala Asp Ser Val Lys Gly
1               5                   10                  15


<210>    94
<211>    16
<212>    PRT
<213>    Homo sapiens

<400>    94
Tyr Ile Tyr His Ser Gly Ser Thr Tyr Tyr Asn Pro Ser Leu Lys Ser
1               5                   10                  15


<210>    95
<211>    16
<212>    PRT
<213>    Homo sapiens

<400>    95
Tyr Ile Tyr Tyr Ser Gly Ser Thr Asn Tyr Asn Pro Ser Leu Lys Ser
1               5                   10                  15


<210>    96
<211>    16
<212>    PRT
<213>    Homo sapiens

<400>    96
Tyr Ile Tyr Tyr Ser Gly Ser Thr Tyr Tyr Asn Pro Ser Leu Lys Ser
1               5                   10                  15


<210>    97
<211>    17
<212>    PRT
<213>    Homo sapiens

<400>    97
Ala Ile Ser Gly Ser Gly Gly Ser Thr Tyr Tyr Ala Asp Ser Val Lys
1               5                   10                  15

Gly


<210>    98
<211>    17
<212>    PRT
<213>    Homo sapiens

<400>    98
Ala Ile Ser Ser Asn Gly Gly Ser Thr Tyr Tyr Ala Asn Ser Val Lys
1               5                   10                  15

Gly

<210>    99
<211>    17
<212>    PRT
<213>    Homo sapiens

<400>    99
Gly Phe Asp Pro Glu Asp Gly Glu Thr Ile Tyr Ala Gln Lys Phe Gln
1               5                   10                  15

Gly

<210>    100
<211>    17
<212>    PRT
<213>    Homo sapiens

<400>    100
Gly Ile Ile Pro Ile Phe Gly Thr Ala Asn Tyr Ala Gln Lys Phe Gln
1               5                   10                  15

Gly

<210>    101
<211>    17
<212>    PRT
<213>    Homo sapiens

<400>    101
Gly Ile Asn Trp Asn Gly Gly Ser Thr Gly Tyr Ala Asp Ser Val Lys
1               5                   10                  15

Gly

<210>    102
<211>    17
<212>    PRT
<213>    Homo sapiens

<400>    102
Gly Ile Ser Trp Asn Ser Gly Ser Ile Gly Tyr Ala Asp Ser Val Lys
1               5                   10                  15

Gly

<210>    103
<211>    17
<212>    PRT
<213>    Homo sapiens

<400>    103
Ile Ile Asn Pro Ser Gly Gly Ser Thr Ser Tyr Ala Gln Lys Phe Gln
1               5                   10                  15

Gly

<210> 104
<211> 17
<212> PRT
<213> Homo sapiens

<400> 104
Ile Ile Tyr Pro Gly Asp Ser Asp Thr Arg Tyr Ser Pro Ser Phe Gln
1               5                   10                  15

Gly

<210> 105
<211> 17
<212> PRT
<213> Homo sapiens

<400> 105
Leu Ile Ser Trp Asp Gly Gly Ser Thr Tyr Tyr Ala Asp Ser Val Lys
1               5                   10                  15

Gly

<210> 106
<211> 17
<212> PRT
<213> Homo sapiens

<400> 106
Leu Val Asp Pro Glu Asp Gly Glu Thr Ile Tyr Ala Glu Lys Phe Gln
1               5                   10                  15

Gly

<210> 107
<211> 17
<212> PRT
<213> Homo sapiens

<400> 107
Asn Ile Lys Gln Asp Gly Ser Glu Lys Tyr Tyr Val Asp Ser Val Lys
1               5                   10                  15

Gly

<210> 108
<211> 17
<212> PRT
<213> Homo sapiens

<400> 108
Arg Ile Asp Pro Ser Asp Ser Tyr Thr Asn Tyr Ser Pro Ser Phe Gln

1               5                   10                  15

Gly

<210>   109
<211>   17
<212>   PRT
<213>   Homo sapiens

<400>   109
Arg Ile Asn Ser Asp Gly Ser Ser Thr Ser Tyr Ala Asp Ser Val Lys
1               5                   10                  15

Gly

<210>   110
<211>   17
<212>   PRT
<213>   Homo sapiens

<400>   110
Ser Ile Ser Ser Ser Ser Ser Tyr Ile Tyr Tyr Ala Asp Ser Val Lys
1               5                   10                  15

Gly

<210>   111
<211>   17
<212>   PRT
<213>   Homo sapiens

<400>   111
Val Ile Ser Tyr Asp Gly Ser Asn Lys Tyr Tyr Ala Asp Ser Val Lys
1               5                   10                  15

Gly

<210>   112
<211>   17
<212>   PRT
<213>   Homo sapiens

<400>   112
Val Ile Trp Tyr Asp Gly Ser Asn Lys Tyr Tyr Ala Asp Ser Val Lys
1               5                   10                  15

Gly

<210>   113
<211>   17
<212>   PRT
<213>   Homo sapiens

<400>    113
Trp Ile Asn Ala Gly Asn Gly Asn Thr Lys Tyr Ser Gln Lys Phe Gln
1               5                   10                  15

Gly

<210>    114
<211>    17
<212>    PRT
<213>    Homo sapiens

<400>    114
Trp Ile Asn Pro Asn Ser Gly Gly Thr Asn Tyr Ala Gln Lys Phe Gln
1               5                   10                  15

Gly

<210>    115
<211>    17
<212>    PRT
<213>    Homo sapiens

<400>    115
Trp Ile Asn Thr Asn Thr Gly Asn Pro Thr Tyr Ala Gln Gly Phe Thr
1               5                   10                  15

Gly

<210>    116
<211>    17
<212>    PRT
<213>    Homo sapiens

<400>    116
Trp Ile Ser Ala Tyr Asn Gly Asn Thr Asn Tyr Ala Gln Lys Leu Gln
1               5                   10                  15

Gly

<210>    117
<211>    17
<212>    PRT
<213>    Homo sapiens

<400>    117
Trp Ile Thr Pro Phe Asn Gly Asn Thr Asn Tyr Ala Gln Lys Phe Gln
1               5                   10                  15

Asp

<210>    118
<211>    17
<212>    PRT

<213>    Homo sapiens

<400>    118
Trp Ile Val Val Gly Ser Gly Asn Thr Asn Tyr Ala Gln Lys Phe Gln
1               5                   10                  15

Glu

<210>    119
<211>    17
<212>    PRT
<213>    Homo sapiens

<400>    119
Trp Met Asn Pro Asn Ser Gly Asn Thr Gly Tyr Ala Gln Lys Phe Gln
1               5                   10                  15

Gly

<210>    120
<211>    17
<212>    PRT
<213>    Homo sapiens

<400>    120
Tyr Ile Ser Ser Ser Gly Ser Thr Ile Tyr Tyr Ala Asp Ser Val Lys
1               5                   10                  15

Gly

<210>    121
<211>    17
<212>    PRT
<213>    Homo sapiens

<400>    121
Tyr Ile Ser Ser Ser Ser Ser Thr Ile Tyr Tyr Ala Asp Ser Val Lys
1               5                   10                  15

Gly

<210>    122
<211>    11
<212>    PRT
<213>    Homo sapiens

<400>    122
Lys Ala Ser Gln Asp Ile Asp Asp Asp Met Asn
1               5                   10

<210>    123
<211>    11
<212>    PRT
<213>    Homo sapiens

<400>    123
Gln Ala Ser Glu Gly Ile Gly Asn Tyr Leu Tyr
1               5                   10

<210>    124
<211>    11
<212>    PRT
<213>    Homo sapiens

<400>    124
Gln Ala Ser Gln Asp Ile Ser Asn Tyr Leu Asn
1               5                   10

<210>    125
<211>    11
<212>    PRT
<213>    Homo sapiens

<400>    125
Arg Ala Arg Gln Gly Ile Ser Asn Tyr Leu Ala
1               5                   10

<210>    126
<211>    11
<212>    PRT
<213>    Homo sapiens

<400>    126
Arg Ala Ser Gln Gly Ile Arg Asn Asp Leu Gly
1               5                   10

<210>    127
<211>    11
<212>    PRT
<213>    Homo sapiens

<400>    127
Arg Ala Ser Gln Gly Ile Ser Asn Tyr Leu Ala
1               5                   10

<210>    128
<211>    11
<212>    PRT
<213>    Homo sapiens

<400>    128
Arg Ala Ser Gln Gly Ile Ser Ser Ala Leu Ala
1               5                   10

<210>    129
<211>    11
<212>    PRT
<213>    Homo sapiens

<400>    129
Arg Ala Ser Gln Gly Ile Ser Ser Trp Leu Ala

1                 5                   10


<210>    130
<211>    11
<212>    PRT
<213>    Homo sapiens

<400>    130
Arg Ala Ser Gln Gly Ile Ser Ser Tyr Leu Ala
1                 5                   10


<210>    131
<211>    11
<212>    PRT
<213>    Homo sapiens

<400>    131
Arg Ala Ser Gln Gly Val Ser Ser Tyr Leu Ala
1                 5                   10


<210>    132
<211>    11
<212>    PRT
<213>    Homo sapiens

<400>    132
Arg Ala Ser Gln Ser Ile Gly Ser Ser Leu His
1                 5                   10


<210>    133
<211>    11
<212>    PRT
<213>    Homo sapiens

<400>    133
Arg Ala Ser Gln Ser Ile Ser Ser Trp Leu Ala
1                 5                   10


<210>    134
<211>    11
<212>    PRT
<213>    Homo sapiens

<400>    134
Arg Ala Ser Gln Ser Ile Ser Ser Tyr Leu Asn
1                 5                   10


<210>    135
<211>    11
<212>    PRT
<213>    Homo sapiens

<400>    135
Arg Ala Ser Gln Ser Val Ser Ser Asn Leu Ala
1                 5                   10

<210>    136
<211>    11
<212>    PRT
<213>    Homo sapiens

<400>    136
Arg Ala Ser Gln Ser Val Ser Ser Tyr Leu Ala
1               5                   10


<210>    137
<211>    11
<212>    PRT
<213>    Homo sapiens

<400>    137
Arg Met Ser Gln Gly Ile Ser Ser Tyr Leu Ala
1               5                   10


<210>    138
<211>    11
<212>    PRT
<213>    Homo sapiens

<400>    138
Trp Ala Ser Gln Gly Ile Ser Ser Tyr Leu Ala
1               5                   10


<210>    139
<211>    12
<212>    PRT
<213>    Homo sapiens

<400>    139
Gly Ala Ser Gln Ser Val Ser Ser Ser Tyr Leu Ala
1               5                   10


<210>    140
<211>    12
<212>    PRT
<213>    Homo sapiens

<400>    140
Arg Ala Ser Gln Ser Val Ser Ser Ser Tyr Leu Ala
1               5                   10


<210>    141
<211>    12
<212>    PRT
<213>    Homo sapiens

<400>    141
Arg Ala Ser Gln Ser Val Ser Ser Ser Tyr Leu Ser
1               5                   10


<210>    142
<211>    16
<212>    PRT

<213>    Homo sapiens

<400>    142
Lys Ser Ser Gln Ser Leu Leu His Ser Asp Gly Lys Thr Tyr Leu Tyr
1               5                   10                  15


<210>    143
<211>    16
<212>    PRT
<213>    Homo sapiens

<400>    143
Arg Ser Ser Gln Ser Leu Leu His Ser Asn Gly Tyr Asn Tyr Leu Asp
1               5                   10                  15


<210>    144
<211>    16
<212>    PRT
<213>    Homo sapiens

<400>    144
Arg Ser Ser Gln Ser Leu Val His Ser Asp Gly Asn Thr Tyr Leu Ser
1               5                   10                  15


<210>    145
<211>    16
<212>    PRT
<213>    Homo sapiens

<400>    145
Arg Ser Ser Gln Ser Leu Val Tyr Ser Asp Gly Asn Thr Tyr Leu Asn
1               5                   10                  15


<210>    146
<211>    7
<212>    PRT
<213>    Homo sapiens

<400>    146
Ala Ala Ser Ser Leu Gln Ser
1               5


<210>    147
<211>    7
<212>    PRT
<213>    Homo sapiens

<400>    147
Ala Ala Ser Thr Leu Gln Ser
1               5


<210>    148
<211>    7
<212>    PRT
<213>    Homo sapiens

<400>    148

Asp Ala Ser Asn Leu Glu Thr
1               5

<210>   149
<211>   7
<212>   PRT
<213>   Homo sapiens

<400>   149
Asp Ala Ser Asn Arg Ala Thr
1               5

<210>   150
<211>   7
<212>   PRT
<213>   Homo sapiens

<400>   150
Asp Ala Ser Ser Leu Glu Ser
1               5

<210>   151
<211>   7
<212>   PRT
<213>   Homo sapiens

<400>   151
Asp Ala Ser Ser Arg Ala Thr
1               5

<210>   152
<211>   7
<212>   PRT
<213>   Homo sapiens

<400>   152
Glu Ala Thr Thr Leu Val Pro
1               5

<210>   153
<211>   7
<212>   PRT
<213>   Homo sapiens

<400>   153
Glu Val Ser Asn Arg Phe Ser
1               5

<210>   154
<211>   7
<212>   PRT
<213>   Homo sapiens

<400>   154
Glu Val Ser Ser Arg Phe Ser
1               5

<210>    155
<211>    7
<212>    PRT
<213>    Homo sapiens

<400>    155
Gly Ala Ser Ser Arg Ala Thr
1               5

<210>    156
<211>    7
<212>    PRT
<213>    Homo sapiens

<400>    156
Gly Ala Ser Thr Arg Ala Thr
1               5

<210>    157
<211>    7
<212>    PRT
<213>    Homo sapiens

<400>    157
Lys Ile Ser Asn Arg Phe Ser
1               5

<210>    158
<211>    7
<212>    PRT
<213>    Homo sapiens

<400>    158
Lys Val Ser Asn Arg Asp Ser
1               5

<210>    159
<211>    7
<212>    PRT
<213>    Homo sapiens

<400>    159
Lys Val Ser Asn Trp Asp Ser
1               5

<210>    160
<211>    7
<212>    PRT
<213>    Homo sapiens

<400>    160
Leu Gly Ser Asn Arg Ala Ser
1               5

<210>    161
<211>    7

<212>    PRT
<213>    Homo sapiens

<400>    161
Thr Leu Ser Tyr Arg Ala Ser
1               5


<210>    162
<211>    7
<212>    PRT
<213>    Homo sapiens

<400>    162
Trp Ala Ser Thr Arg Glu Ser
1               5


<210>    163
<211>    7
<212>    PRT
<213>    Homo sapiens

<400>    163
Tyr Ala Ser Gln Ser Phe Ser
1               5


<210>    164
<211>    7
<212>    PRT
<213>    Homo sapiens

<400>    164
Tyr Ala Ser Gln Ser Ile Ser
1               5


<210>    165
<211>    7
<212>    PRT
<213>    Homo sapiens

<400>    165
Tyr Ala Ser Ser Leu Gln Ser
1               5


<210>    166
<211>    11
<212>    PRT
<213>    Homo sapiens

<400>    166
Gly Gly Asn Asn Ile Gly Ser Lys Asn Val His
1               5                   10


<210>    167
<211>    11
<212>    PRT
<213>    Homo sapiens

<400>    167
Gly Gly Asn Asn Ile Gly Ser Lys Ser Val His
1               5                   10

<210>    168
<211>    11
<212>    PRT
<213>    Homo sapiens

<400>    168
Gln Gly Asp Ser Leu Arg Ser Tyr Tyr Ala Ser
1               5                   10

<210>    169
<211>    11
<212>    PRT
<213>    Homo sapiens

<400>    169
Ser Gly Asp Ala Leu Pro Lys Lys Tyr Ala Tyr
1               5                   10

<210>    170
<211>    11
<212>    PRT
<213>    Homo sapiens

<400>    170
Ser Gly Asp Ala Leu Pro Lys Gln Tyr Ala Tyr
1               5                   10

<210>    171
<211>    11
<212>    PRT
<213>    Homo sapiens

<400>    171
Ser Gly Asp Lys Leu Gly Asp Lys Tyr Ala Cys
1               5                   10

<210>    172
<211>    11
<212>    PRT
<213>    Homo sapiens

<400>    172
Ser Gly Asp Val Leu Ala Lys Lys Tyr Ala Arg
1               5                   10

<210>    173
<211>    11
<212>    PRT
<213>    Homo sapiens

<400>    173
Ser Gly Asp Val Leu Gly Glu Asn Tyr Ala Asp
1               5                   10

<210>    174
<211>    11
<212>    PRT
<213>    Homo sapiens

<400>    174
Ser Gly Glu Ala Leu Pro Lys Lys Tyr Ala Tyr
1               5                   10


<210>    175
<211>    13
<212>    PRT
<213>    Homo sapiens

<400>    175
Ser Gly Ser Ser Ser Asn Ile Gly Asn Asn Ala Val Asn
1               5                   10


<210>    176
<211>    13
<212>    PRT
<213>    Homo sapiens

<400>    176
Ser Gly Ser Ser Ser Asn Ile Gly Asn Asn Tyr Val Ser
1               5                   10


<210>    177
<211>    13
<212>    PRT
<213>    Homo sapiens

<400>    177
Ser Gly Ser Ser Ser Asn Ile Gly Ser Asn Thr Val Asn
1               5                   10


<210>    178
<211>    13
<212>    PRT
<213>    Homo sapiens

<400>    178
Ser Gly Ser Ser Ser Asn Ile Gly Ser Asn Tyr Val Tyr
1               5                   10


<210>    179
<211>    13
<212>    PRT
<213>    Homo sapiens

<400>    179
Thr Arg Ser Ser Gly Ser Ile Ala Ser Asn Tyr Val Gln
1               5                   10


<210>    180

<211>    14
<212>    PRT
<213>    Homo sapiens

<400>    180
Ala Ser Ser Thr Gly Ala Val Thr Ser Gly Tyr Tyr Pro Asn
1               5                   10


<210>    181
<211>    14
<212>    PRT
<213>    Homo sapiens

<400>    181
Gly Leu Ser Ser Gly Ser Val Ser Thr Ser Tyr Tyr Pro Ser
1               5                   10


<210>    182
<211>    14
<212>    PRT
<213>    Homo sapiens

<400>    182
Gly Ser Ser Thr Gly Ala Val Thr Ser Gly His Tyr Pro Tyr
1               5                   10


<210>    183
<211>    14
<212>    PRT
<213>    Homo sapiens

<400>    183
Met Leu Ser Ser Gly Phe Ser Val Gly Asp Phe Trp Ile Arg
1               5                   10


<210>    184
<211>    14
<212>    PRT
<213>    Homo sapiens

<400>    184
Thr Gly Ser Ser Ser Asn Ile Gly Ala Gly Tyr Asp Val His
1               5                   10


<210>    185
<211>    14
<212>    PRT
<213>    Homo sapiens

<400>    185
Thr Gly Thr Ser Ser Asp Val Gly Gly Tyr Asn Tyr Val Ser
1               5                   10


<210>    186
<211>    14
<212>    PRT
<213>    Homo sapiens

<400>    186
Thr Gly Thr Ser Ser Asp Val Gly Ser Tyr Asn Leu Val Ser
1               5                   10

<210>    187
<211>    14
<212>    PRT
<213>    Homo sapiens

<400>    187
Thr Gly Thr Ser Ser Asp Val Gly Ser Tyr Asn Arg Val Ser
1               5                   10

<210>    188
<211>    14
<212>    PRT
<213>    Homo sapiens

<400>    188
Thr Leu Pro Ser Asp Ile Asn Val Gly Ser Tyr Asn Ile Tyr
1               5                   10

<210>    189
<211>    14
<212>    PRT
<213>    Homo sapiens

<400>    189
Thr Leu Arg Ser Gly Ile Asn Val Gly Thr Tyr Arg Ile Tyr
1               5                   10

<210>    190
<211>    7
<212>    PRT
<213>    Homo sapiens

<400>    190
Asp Asn Asn Lys Arg Pro Ser
1               5

<210>    191
<211>    7
<212>    PRT
<213>    Homo sapiens

<400>    191
Asp Thr Ser Asn Lys His Ser
1               5

<210>    192
<211>    7
<212>    PRT
<213>    Homo sapiens

<400>    192
Asp Val Ser Lys Arg Pro Ser

1                   5


<210>    193
<211>    7
<212>    PRT
<213>    Homo sapiens

<400>    193
Glu Asp Asn Gln Arg Pro Ser
1                   5


<210>    194
<211>    7
<212>    PRT
<213>    Homo sapiens

<400>    194
Glu Asp Ser Glu Arg Tyr Pro
1                   5


<210>    195
<211>    7
<212>    PRT
<213>    Homo sapiens

<400>    195
Glu Asp Ser Lys Arg Pro Ser
1                   5


<210>    196
<211>    7
<212>    PRT
<213>    Homo sapiens

<400>    196
Glu Val Ser Lys Arg Pro Ser
1                   5


<210>    197
<211>    7
<212>    PRT
<213>    Homo sapiens

<400>    197
Glu Val Ser Asn Arg Pro Ser
1                   5


<210>    198
<211>    7
<212>    PRT
<213>    Homo sapiens

<400>    198
Gly Lys Asn Asn Arg Pro Ser
1                   5

<210>    199
<211>    7
<212>    PRT
<213>    Homo sapiens

<400>    199
Gly Asn Ser Asn Arg Pro Ser
1               5

<210>    200
<211>    7
<212>    PRT
<213>    Homo sapiens

<400>    200
Lys Asp Ser Glu Arg Pro Ser
1               5

<210>    201
<211>    7
<212>    PRT
<213>    Homo sapiens

<400>    201
Gln Asp Ser Lys Arg Pro Ser
1               5

<210>    202
<211>    7
<212>    PRT
<213>    Homo sapiens

<400>    202
Arg Asp Ser Asn Arg Pro Ser
1               5

<210>    203
<211>    7
<212>    PRT
<213>    Homo sapiens

<400>    203
Arg Asn Asn Asn Arg Pro Ser
1               5

<210>    204
<211>    7
<212>    PRT
<213>    Homo sapiens

<400>    204
Arg Asn Asn Gln Arg Pro Ser
1               5

<210>    205
<211>    7
<212>    PRT

<213>    Homo sapiens

<400>    205
Ser Asn Asn Gln Arg Pro Ser
1               5


<210>    206
<211>    7
<212>    PRT
<213>    Homo sapiens

<400>    206
Ser Thr Asn Thr Arg Ser Ser
1               5


<210>    207
<211>    7
<212>    PRT
<213>    Homo sapiens

<400>    207
Ser Thr Ser Asn Lys His Ser
1               5


<210>    208
<211>    7
<212>    PRT
<213>    Homo sapiens

<400>    208
Tyr Asp Asp Leu Leu Pro Ser
1               5


<210>    209
<211>    7
<212>    PRT
<213>    Homo sapiens

<400>    209
Tyr Asp Ser Asp Arg Pro Ser
1               5


<210>    210
<211>    7
<212>    PRT
<213>    Homo sapiens

<400>    210
His Gln Ser Ser Ser Leu Pro
1               5


<210>    211
<211>    7
<212>    PRT
<213>    Homo sapiens

<400>    211

Leu Gln Asp Tyr Asn Tyr Pro
1               5

<210>   212
<211>   7
<212>   PRT
<213>   Homo sapiens

<400>   212
Leu Gln His Asp Asn Phe Pro
1               5

<210>   213
<211>   7
<212>   PRT
<213>   Homo sapiens

<400>   213
Leu Gln His Asn Ser Tyr Pro
1               5

<210>   214
<211>   7
<212>   PRT
<213>   Homo sapiens

<400>   214
Met Gln Ala Leu Gln Thr Pro
1               5

<210>   215
<211>   7
<212>   PRT
<213>   Homo sapiens

<400>   215
Met Gln Ala Thr Gln Phe Pro
1               5

<210>   216
<211>   7
<212>   PRT
<213>   Homo sapiens

<400>   216
Met Gln Gly Ile His Leu Pro
1               5

<210>   217
<211>   7
<212>   PRT
<213>   Homo sapiens

<400>   217
Met Gln Gly Thr His Trp Pro
1               5

<210>    218
<211>    7
<212>    PRT
<213>    Homo sapiens

<400>    218
Met Gln Arg Ile Glu Phe Pro
1               5

<210>    219
<211>    7
<212>    PRT
<213>    Homo sapiens

<400>    219
Met Gln Ser Ile Gln Leu Pro
1               5

<210>    220
<211>    7
<212>    PRT
<213>    Homo sapiens

<400>    220
Gln Lys Tyr Asn Ser Ala Pro
1               5

<210>    221
<211>    7
<212>    PRT
<213>    Homo sapiens

<400>    221
Gln Gln Ala Asn Ser Phe Pro
1               5

<210>    222
<211>    7
<212>    PRT
<213>    Homo sapiens

<400>    222
Gln Gln Asp Tyr Asn Leu Pro
1               5

<210>    223
<211>    7
<212>    PRT
<213>    Homo sapiens

<400>    223
Gln Gln Phe Asn Ser Tyr Pro
1               5

<210>    224
<211>    7

<212>    PRT
<213>    Homo sapiens

<400>    224
Gln Gln Gly Asn Lys His Pro
1               5


<210>    225
<211>    7
<212>    PRT
<213>    Homo sapiens

<400>    225
Gln Gln Leu Asn Ser Tyr Pro
1               5


<210>    226
<211>    7
<212>    PRT
<213>    Homo sapiens

<400>    226
Gln Gln Arg Ser Asn Trp His
1               5


<210>    227
<211>    7
<212>    PRT
<213>    Homo sapiens

<400>    227
Gln Gln Arg Ser Asn Trp Pro
1               5


<210>    228
<211>    7
<212>    PRT
<213>    Homo sapiens

<400>    228
Gln Gln Ser Tyr Ser Thr Pro
1               5


<210>    229
<211>    7
<212>    PRT
<213>    Homo sapiens

<400>    229
Gln Gln Tyr Asp Asn Leu Pro
1               5


<210>    230
<211>    7
<212>    PRT
<213>    Homo sapiens

<400>    230
Gln Gln Tyr Gly Ser Ser Pro
1               5

<210>    231
<211>    7
<212>    PRT
<213>    Homo sapiens

<400>    231
Gln Gln Tyr Asn Asn Trp Pro
1               5

<210>    232
<211>    7
<212>    PRT
<213>    Homo sapiens

<400>    232
Gln Gln Tyr Asn Ser Tyr Pro
1               5

<210>    233
<211>    7
<212>    PRT
<213>    Homo sapiens

<400>    233
Gln Gln Tyr Asn Ser Tyr Ser
1               5

<210>    234
<211>    7
<212>    PRT
<213>    Homo sapiens

<400>    234
Gln Gln Tyr Tyr Ser Phe Pro
1               5

<210>    235
<211>    7
<212>    PRT
<213>    Homo sapiens

<400>    235
Gln Gln Tyr Tyr Ser Thr Pro
1               5

<210>    236
<211>    7
<212>    PRT
<213>    Homo sapiens

<400>    236
Gln Gln Tyr Tyr Ser Tyr Pro
1               5

<210>    237
<211>    9
<212>    PRT
<213>    Homo sapiens

<400>    237
Ala Ala Trp Asp Asp Ser Leu Asn Gly
1               5

<210>    238
<211>    9
<212>    PRT
<213>    Homo sapiens

<400>    238
Ala Ala Trp Asp Asp Ser Leu Ser Gly
1               5

<210>    239
<211>    9
<212>    PRT
<213>    Homo sapiens

<400>    239
Cys Ser Tyr Ala Gly Ser Ser Thr Phe
1               5

<210>    240
<211>    9
<212>    PRT
<213>    Homo sapiens

<400>    240
Cys Ser Tyr Ala Gly Ser Tyr Thr Phe
1               5

<210>    241
<211>    9
<212>    PRT
<213>    Homo sapiens

<400>    241
Gly Thr Trp Asp Ser Ser Leu Ser Ala
1               5

<210>    242
<211>    9
<212>    PRT
<213>    Homo sapiens

<400>    242
Leu Ser Ala Asp Ser Ser Gly Thr Tyr
1               5

<210>    243

<211>    9
<212>    PRT
<213>    Homo sapiens

<400>    243
Asn Ser Arg Asp Ser Ser Gly Asn His
1               5

<210>    244
<211>    8
<212>    PRT
<213>    Homo sapiens

<400>    244
Gln Ala Trp Asp Ser Ser Thr Ala
1               5

<210>    245
<211>    9
<212>    PRT
<213>    Homo sapiens

<400>    245
Gln Ser Ala Asp Ser Ser Gly Thr Tyr
1               5

<210>    246
<211>    9
<212>    PRT
<213>    Homo sapiens

<400>    246
Gln Ser Tyr Asp Ser Ser Leu Ser Gly
1               5

<210>    247
<211>    9
<212>    PRT
<213>    Homo sapiens

<400>    247
Gln Val Trp Asp Ser Ser Ser Asp His
1               5

<210>    248
<211>    8
<212>    PRT
<213>    Homo sapiens

<400>    248
Gln Val Trp Asp Ser Ser Thr Ala
1               5

<210>    249
<211>    9
<212>    PRT
<213>    Homo sapiens

<400>    249
Ser Leu Tyr Thr Ser Ser Ser Thr Phe
1               5

<210>    250
<211>    9
<212>    PRT
<213>    Homo sapiens

<400>    250
Ser Ser Tyr Ala Gly Ser Asn Asn Phe
1               5

<210>    251
<211>    9
<212>    PRT
<213>    Homo sapiens

<400>    251
Ser Ser Tyr Thr Ser Ser Ser Thr Leu
1               5

<210>    252
<211>    9
<212>    PRT
<213>    Homo sapiens

<400>    252
Tyr Ser Thr Asp Ser Ser Gly Asn His
1               5

## Description

### Technical Field

The present invention relates to a method for preparing a novel antibody library and a library prepared thereby.

### Background Art

A phage display technique is a technique in which bacteriophage using a bacterium as a host is genetically engineered to connect a genotype (gene) and a phenotype (protein) through a single phage particle. In this case, the gene as a genotype is inserted into a part of a phage gene, and the protein as a phenotype is displayed on a surface of the phage particle containing a gene of the protein. The physical combination between genotype and phenotype, which is a very important concept in protein engineering, enables replication, amplification, analysis, and engineering of proteins by allowing one to easily identify a gene of protein clones selected by a property exhibited as a phenotype.

An antibody is an example to which, particularly, a phage display technique is very usefully applied. When an antibody library having very high diversity is displayed on phage surface and allowed to bind to a surface-adsorbed antigen, genes of antibody clones selectively binding to the antigen can be obtained. This method is very effective in obtaining antibodies without using experimental animals, and has very high applicability in the development of therapeutic antibody drugs with low immunogenicity in human subjects since, particularly, antibodies to given antigens can be obtained. The quality of the library is important for obtaining good antibodies with high binding affinity, and particularly, the size and functional diversity of the library and the quality of the clones constituting the library are important.

The size of a library is one of the most important factors that determine the quality of antibodies selected from the library. The antigen binding site of an antibody library has random diversity that is not theoretically involved biased towards or against any particular antigen, and an antibody selectively binding to a particular antigen by pure chance can be isolated from the random diversity. Therefore, as the size of the library increases, i.e., as the number of different antibodies in the library increases, the likelihood of finding an antibody having high selectivity and affinity by chance is increased. Antibody libraries are generally considered to need a size of at least 10⁸, and many antibody libraries have a size of about 10⁹ to 10¹¹.

The functional diversity of a library is the percentage of clones that can actually express antibodies among the clones that make up the library. Even though the size of a library is large, low functional diversity decreases the substantial size of the library. The low functional diversity is largely due to errors in DNA synthesis and amplification during library construction. Antibody libraries are constructed through several steps of polymerase chain reaction (PCR), which inevitably results in a low frequency of errors due to the nature of enzymes and reactions, and the accumulation of such errors lowers functional diversity of the final library. Especially, in cases of synthetic libraries, the possibility of introducing errors may be increased due to efficiency problems of the oligonucleotide synthesis reactions. As described above, the problem of functional diversity tends to be more noticeable particularly in synthetic libraries, and most synthetic libraries need to be designed to avoid such a problem.

The quality of individual clones constituting a library, that is, the expression property, stability, immunogenicity, and the like are factors that determine the performance of the antibody library. In antibody engineering perspective, these factors need to be considered during the design phase of the synthetic antibody library construction in order to select high-quality clones from the library. Especially, during the introduction of artificial diversity into existing antibody genes, the generated diversity needs to be designed to have compatibility with the antibody frameworks, while a radical change from an amino acid sequence of a natural antibody poses a risk of impeding compatibility, stability and the like of artificial synthetic antibodies. Therefore, when artificial diversity is designed, efficient simulation of natural diversity is very important in the design and construction of synthetic antibody libraries. In addition, an antibody library composed of antibodies with various sequences may includes sites in which undesired protein modifications, such as glycosylation, oxidation, isomerization, and deamidation, may occur, while these modifications may adversely affect the physical properties and commercial development of the antibodies.

When antibodies are produced in an animal body, antibody sequences having very high diversity are generated through the recombination of tens to hundreds of germline immunoglobulin genes present in the genome, and, among those antibody sequences, antibodies responding to particular antigens are selected. The binding strength of the selected antibodies to antigens is improved through a hypermutation process, finally resulting in mature antibodies. Therefore, the sequence of each mature antibody, especially, the sequences of complementarity determining regions (CDRs) that make direct contact with the antigen are derived from germline gene sequences, while various sequences different from the germline CDR sequences are produced through recombination and mutagenesis. In designing a synthetic antibody library, a construction strategy needs to be established that simulates CDR sequences of natural antibodies generated by such procedures.

Antibody generation methods from natural sources require considerable efforts and time for producing each antibody, and thus, attempts to use synthetic antibody libraries have recently received attention. However, existing synthetic antibody libraries are constructed by randomly synthesizing variation sequences corresponding to CDRs, causing a problem in that the percentage of actually functioning antibodies is low or the efficiency of the library is not ensured.

### Detailed Description of the Invention

### Technical Problem

The present inventors endeavored to construct a high-quality synthetic human antibody library with high functional diversity, which contains antibodies having similar sequences to natural antibodies and retains excellent physical properties, through a fast and efficient procedure. As a result, the present inventors confirmed that a synthetic antibody library containing antibodies having similar sequences to natural antibodies and retaining excellent physical properties was constructed by analyzing CDR sequences of natural antibodies to design artificial sequences and synthesizing them, and therefore, the present inventors completed the present invention.

### Technical Solution

In accordance with an aspect of the present invention, there is provided a method for constructing an antibody library, the method including: individually designing complementarity determining region (CDR) sequences of an antibody; and synthesizing antibodies that include the designed complementarity determining region sequences to construct a library.

In accordance with another aspect of the present invention, there is provided an antibody library constructed by the above method for constructing an antibody library.

### Advantageous Effects

The antibody library constructed according to the present invention contains antibodies having excellent physicochemical properties to a plurality of antigens, and thus can be favorably used as an antibody library having high functional diversity and containing a variety of unique sequences.

### Brief Description of the Drawings

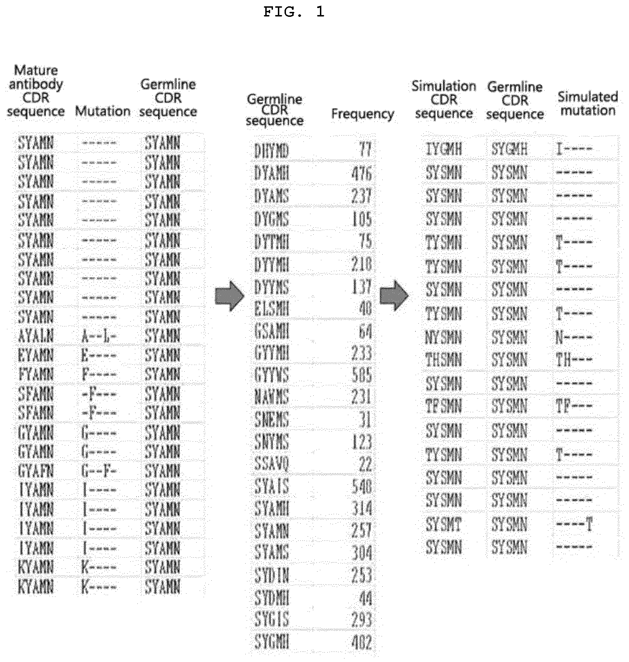

FIG. 1 is a schematic diagram showing a concept of CDR design.

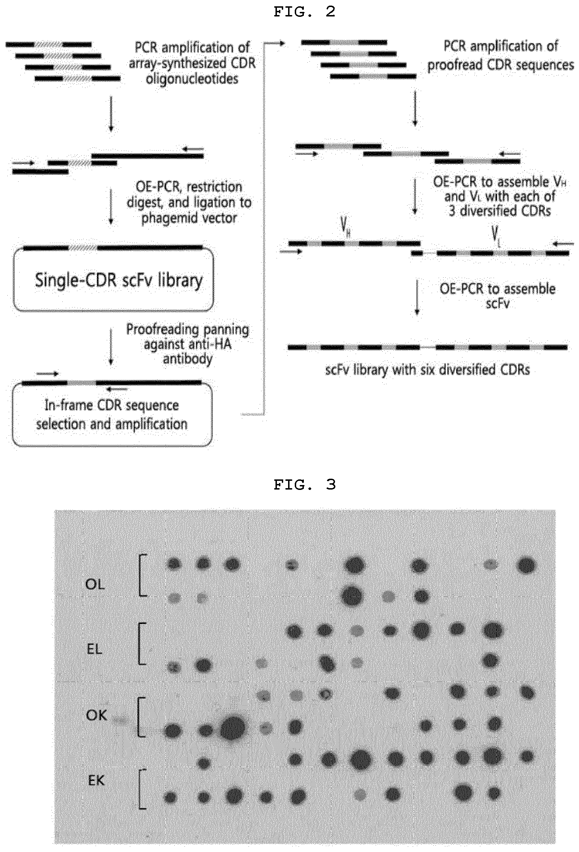

FIG. 2 is a schematic diagram showing a method for constructing an scFv library using six non-combinatorially diversified CDRs. Pools of oligonucleotides with designed CDR sequences were array-synthesized, and amplified by PCR. A single-CDR library (scFv library having only one CDR diversified out of six CDRs) was constructed for each CDR, and panned using anti-HA antibody that binds to HA-tag at the C-terminus of scFv in order to proofread the CDR repertoires for in-frame sequences.
FIG. 3 is a view in which, in order to examine whether scFv is expressed in the antibody library of the present invention, scFv clones before selection are randomly chosen from the library to examine the expression using HA tag. Specifically, 92 clones were randomly chosen, cultured, and induced with IPTG, and the expression of full-length scFv was examined in periplasmic extracts using an anti-HA antibody. Out of 92 clones, 58 clones (63%) were verified to be expressed.

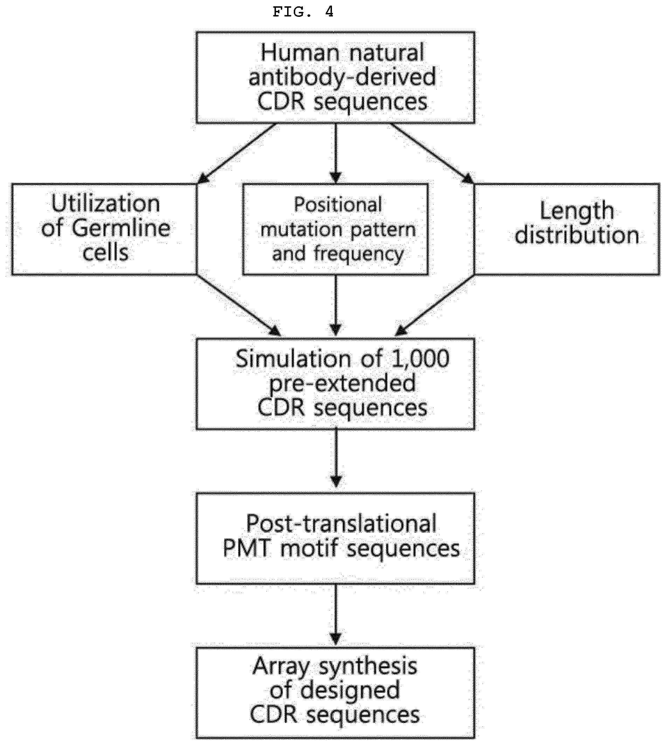

FIG. 4 is a schematic diagram showing steps for constructing an antibody library of the present invention.

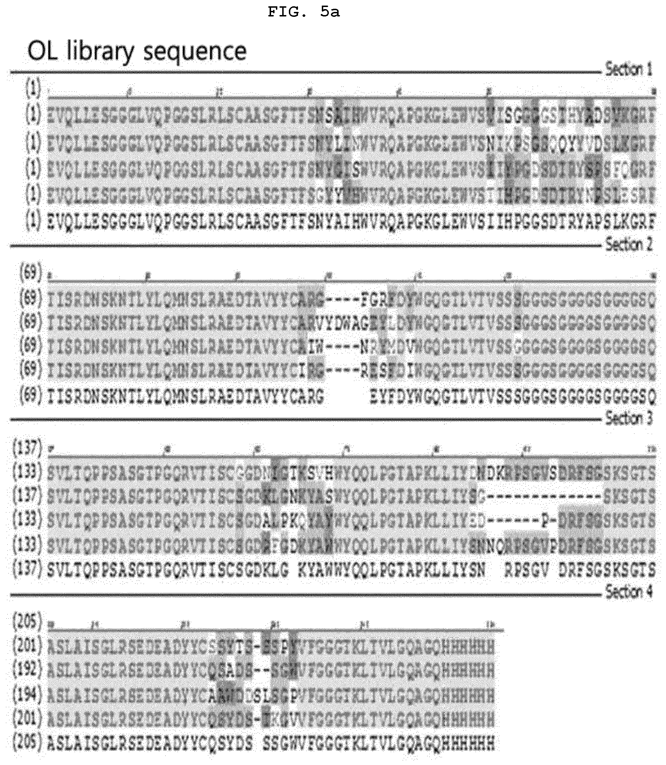

FIG. 5a is a diagram showing the sequencing results of DNA, which was purified after arbitrary clones were selected from OL (odd-lambda) sub-library among the antibody sub-libraries of the present invention and then scFv gene was amplified through PCR.

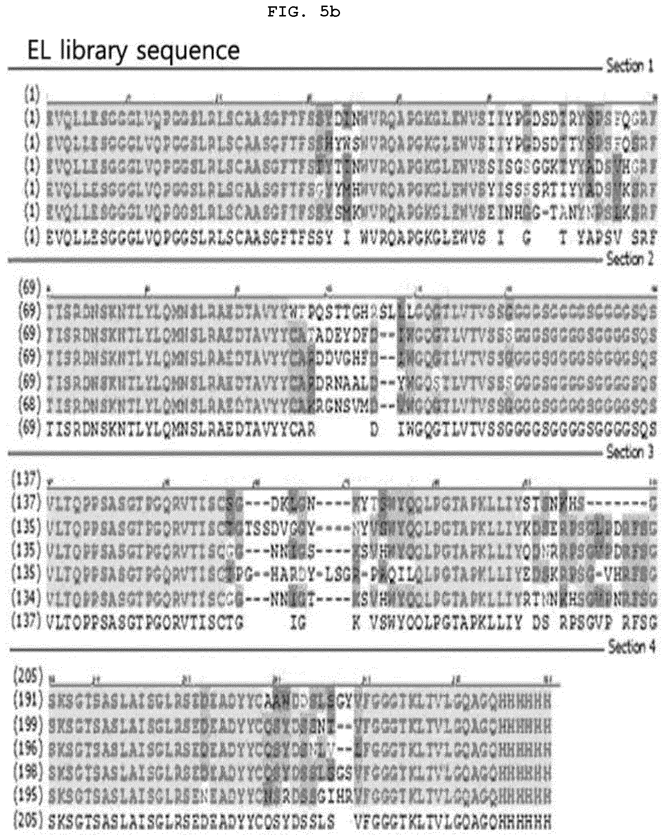

FIG. 5b is a diagram showing the sequencing results of DNA, which was purified after arbitrary clones were selected from EL (even-lambda) sub-library among the antibody sub-libraries of the present invention and then scFv gene was amplified through PCR.

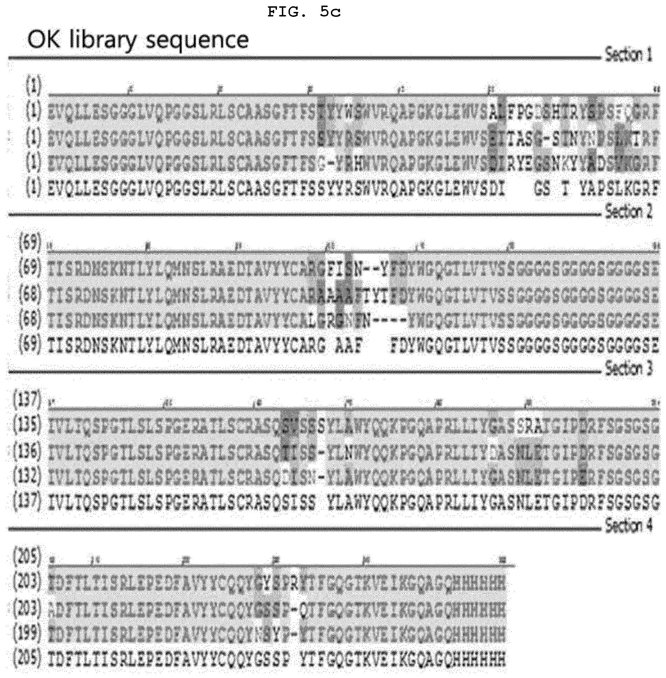

FIG. 5c is a diagram showing the sequencing results of DNA, which was purified after arbitrary clones were selected from OK (odd-kappa) sub-library among the antibody sub-libraries of the present invention and then scFv gene was amplified through PCR.

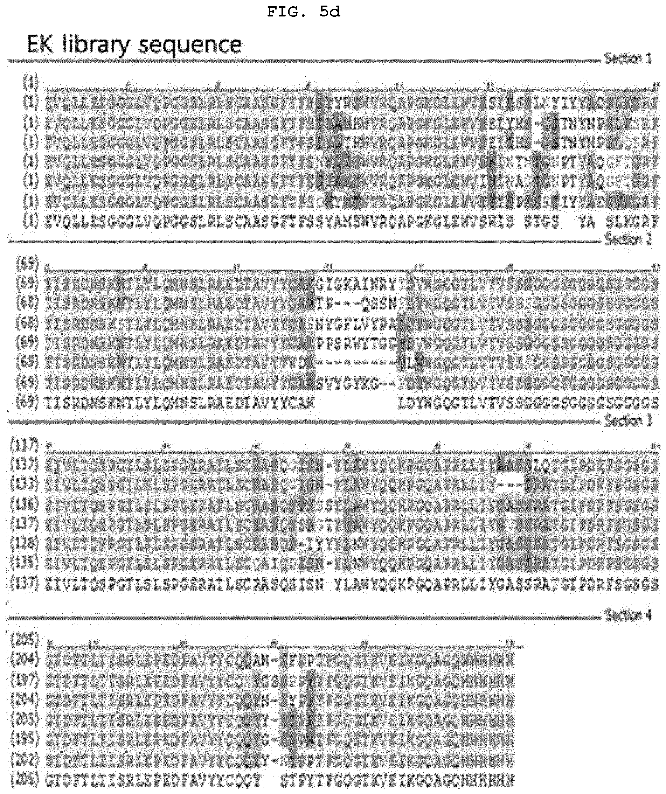

FIG. 5d is a diagram showing the sequencing results of DNA, which was purified after arbitrary clones were selected from EK (even-kappa) sub-library among the antibody sub-libraries of the present invention and then scFv gene was amplified through PCR.

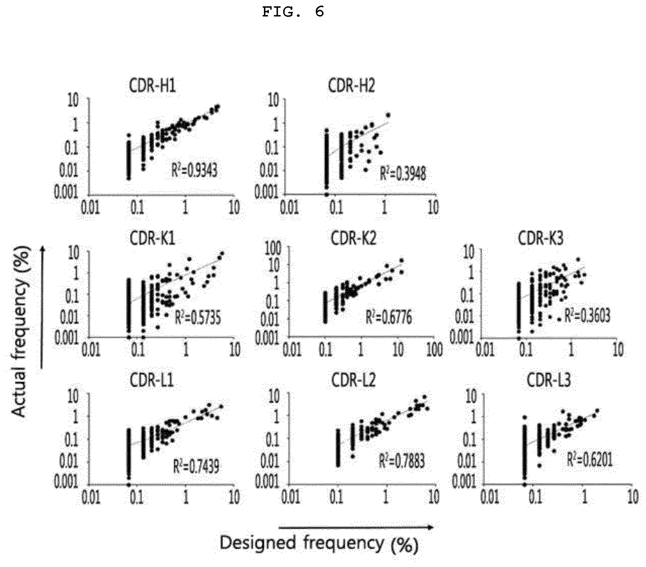

FIG. 6 is a graph showing frequencies of unique CDR sequences in the designed and actual CDR repertoires of the library. The frequencies of occurrence of the NGS-analyzed CDR repertoires of the actual constructed scFv library and each unique CDR sequence in the designed CDR repertoires are shown in XY-distribution plots. Each dot in the plots represents a unique CDR sequence. Meanwhile, CDR-H3 sequences were not analyzed since most of the sequences occur only once in the designed repertoire.

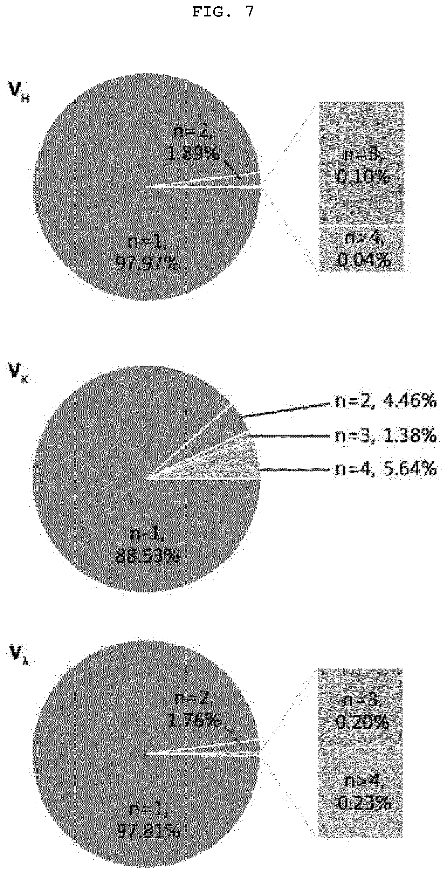

FIG. 7 is a diagram showing variable domain sequence redundancies of the constructed library. The variable domain sequences of the unselected library were obtained through 300 bp paired-end sequencing on Illumina MiSeq platform, and the number of replicates (n) for the variable domain sequences was analyzed. Approximately 98% of V_{H} and V_{λ} and 88.5% of V_{κ} sequences were found only once (n = 1).

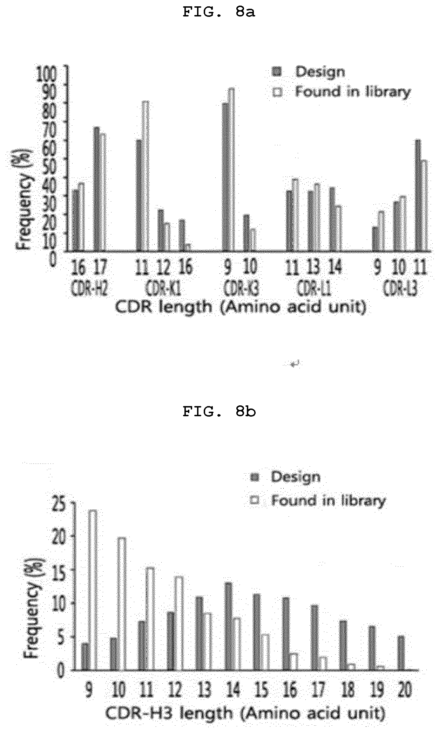

FIG. 8a and 8b are diagrams showing the length distribution of designed CDRs and actual CDRs. In FIG. 8a, CDR-H2, L1 (kappa and lambda), and L3 (kappa and lambda) contain sequences with various lengths. Through a comparision between the length distribution in the designed repertoire and the next generation sequencing (NGS)-analyzed length distribution, the results were obtained indicating that shorter CDRs were preferred in the actual library. In FIG. 8b, the preference for the shorter CDRs was more evident in CDR-H3 which has a wider range of length variation than other CDRs. In both FIG. 8a and FIG. 8b, the blue bars ("Designed") indicate the frequency of each CDR length in the designed repertoire, while the orange bars ("Found in the library") indicate the frequency of each CDR length found from the next generation sequencing (NGS) of the constructed library.

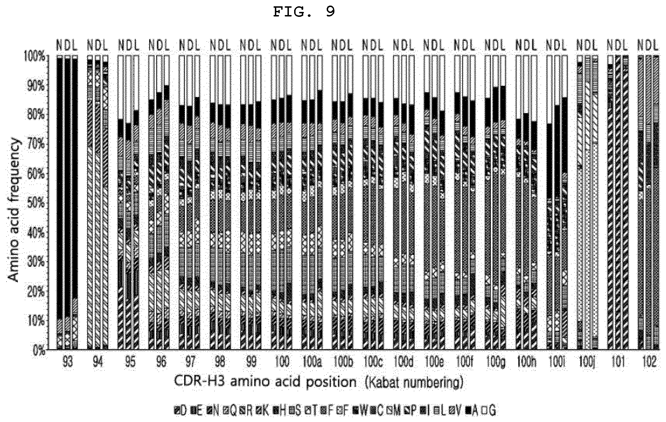

FIG. 9 is a diagram showing the amino acid distribution of CDR-H3. The amino acid distribution of CDR-H3 of the natural human antibodies (N), the designed repertoire (D), and the actually constructed library (L) are shown for each position. Each overlapping bar reflects a total of frequencies of amino acids at each Kabat position of CDR-H3 with different lengths. For all CDR-H3s with different lengths, the last three residues are denoted by 100j, 101, and 102, respectively.

### Best Mode for Carrying Out the Invention

In accordance with an aspect of the present inveniton, there is provided a method for preparing an antibody library, the method comprising: individually designing complementarity determining region (CDR) sequences of antibodies; and synthesizing antibodies comprising the designed complementarity determining region sequences to construct a library.

In one specific embodiment, the present invention provides a method for constructing an antibody library, wherein heavy chain complementarity determining region 1 (CDR-H1), heavy chain complementarity determining region 2 (CDR-H2), heavy chain complementarity determining region 3 (CDR-H3), light chain complementarity determining region 1 (CDR-L1), light chain complementarity determining region 2 (CDR-L2), and light chain complementarity determining region 3 (CDR-L3), which constitute the complementarity determining regions of the antibodies included in the antibody library, have diversity.

Meanwhile, when a light chain complementarity determining region sequence is designed, a corresponding light chain may be a kappa light chain or lambda light chain.

In another specific embodiment, the present inveniton provides a method for constructing an antibody library, wherein in the individual designing of the complementarity determining region sequences, for CDR-H1, CDR-H2, CDR-L1, or CDR-L2, the sequences therefor are designed by simulating i) an utilization frequency of each germline immunoglobulin gene, ii) a frequency of mutation into any 20 amino acid types by somatic hypermutations at each amino acid position, iii) a length distribution frequency of sequences comprising each complementarity determining region, or iv) a frequency of each amino acid at each position calculated by analyzing a combination thereof, of the complementarity determining regions of actual human-derived mature antibodies.

In still another embodiment, the present inveniton provides a method for constructing an antibody library, wherein in the individual designing of the complementarity determining region sequences, for CDR-L3,
a) 7 or 8 amino acid sequences from a N-terminus of the complementarity determining region are designed by simulating i) an utilization frequency of each germline immunoglobulin gene, ii) a frequency of mutation into each of 20 amino acids by somatic hypermutations at each amino acid position, iii) a length distribution frequency of sequences comprising CDR-L3, or iv) a frequency of each amino acid at each position calculated by analyzing a combination thereof, of the complementarity determining region of actual human-derived mature antibodies, and
b) 2 or 3 amino acid sequences from a C-terminus of the complementarity determining region are designed by analyzing and calculating a frequency of each amino acid at each position in the complementarity determining region of actual human-derived mature antibodies, then simulating sequences that reflect the calculated frequencies; and
wherein the CDR-L3 contains 9 to 11 amino acids and the analysis of the frequencies is conducted according to each length, the CDR-L3 sequences being designed based on an analysis result of complementarity determining region CDR-L3 of human-derived mature antibodies, which have the same amino acid lengths as CDR-L3 to be designed.

In still another embodiment, the present inveniton provides a method for constructing an antibody library, wherein, when a light chain complementarity determining region sequence is designed, a corresponding light chain is a kappa light chain or a lambda light chain.

In still another embodment, the present inveniton provides a method for constructing an antibody library, wherein in the individual designing of the complementarity determining region sequences, for CDR-H3,
a) each sequence therefor excluding three amino acids from a C-terminus of the complementarity determining region is designed by using a frequency of each amino acid at each position in the complementarity determining region of actual human-derived mature antibodies, and
b) a 3 amino acid sequence from the C-terminus of the complementarity determining region is designed by analyzing and calculating frequencies of the corresponding 3 amino acid sequences in the complementarity determining region of actual human-derived mature antibodies, then simulating sequences that reflect the calculated frequencies; and
wherein the CDR-H3 contains 9 to 20 amino acids and the analysis of the frequencies is conducted according to each length, the CDR-H3 sequences being designed based on an analysis result of complementarity determining region CDR-H3 of human-derived mature antibodies, which have the same amino acid length as CDR-H3 to be designed.

In still another embodment, the present inveniton provides a method for constructing an antibody library, the method further including, after the designing of the complementarity determining region amino acid sequences, excluding sequences having N-glycosylation, isomerization, deamidation, cleavage, and oxidation motifs from the designed sequences.

In still another embodment, the present inveniton provides a method for constructing an antibody library, the method further including, after the designing of the complementarity determining region amino acid sequences, reverse-translating the designed sequences into a polynucloeotide sequences and then designing an oligonucleotide sequence, in which framework region sequences of variable regions of a human antibody germline gene flanking the complementarity determining region are linked to the 5' and 3' ends of the reverse-translated polynucleotide.

In still another embodment, the present inveniton provides a method for constructing an antibody library, wherein the antibodies include amino acid sequences encoded by VH3-23 (Genebank accession No. Z12347), VK3-A27 (Genebank accession No. X93639), VL1g (GenBank accession No. Z73663), or fragments thereof.

In still another embodiment, the present inveniton provides a method for constructing an antibody library, wherein in the individual designing of the complementarity determining region sequences, when light chain complementarity determinig regions are designed, each CDR is designed for the kappa light chain and the lambda light chain.

In still another embodiment, the present inveniton provides a method for constructing an antibody library, wherein, when each light chain complementarity determinig region is designed for the kappa light chain and the lambda light chain, the kappa light chain CDR is assembled by linkage with a kappa light chain framework region of VK3-A27, and the lambda light chain CDR is assembled by linkage with a lambda light chain framework region of VL1g.

In still another emdbodiment, the present invention provides a method for constructing an antibody library, wherein the antibodies are selected from the group consisting of IgA, IgD, IgE, IgM, IgG, Fc fragments, Fab, Fab', F(ab')₂, scFv, single variable domain antibody, and Fv.

In still another emdbodiment, the present invention provides a method for constructing an antibody library, wherein the method corresponds to at least one of 1) to 6) below:
1) using an amino acid sequence of SEQ ID NO: 54 to SEQ ID NO: 84 as the germline CDR sequence for the heavy chain CDR-H1;
2) using an amino acid sequence of SEQ ID NO: 85 to SEQ ID NO: 121 as the germline CDR sequence for the heavy chain CDR-H2;
3) using a kappa light chain for the germline CDR sequence used for the designing of the light chain CDR and using an amino acid sequence of SEQ ID NO: 122 to 145 as the germline CDR sequence for kappa light chain CDR-L1;
4) using a kappa light chain for the germline CDR sequence used for the designing of the light chain CDR and using an amino acid sequence of SEQ ID NO: 146 to 165 as the germline CDR sequence for kappa light chain CDR-L2;
5) using a lambda light chain for the germline CDR sequence used for the designing of the light chain CDR and using an amino acid sequence of SEQ ID NO: 166 to 189 as the germline CDR sequence for lambda light chain CDR-L1; and
6) using a lambda light chain for the germline CDR sequence used for the designing of the light chain CDR and using an amino acid sequence of SEQ ID NO: 190 to 209 as the germline CDR sequence for lambda light chain CDR-L2.

In still another emdbodiment, the present invention provides a method for constructing an antibody library, wherein the method corresponds to at least one of 1) to 2) below:
1) using a kappa light chain for the germline CDR sequence used for the designing of the light chain CDR and using an amino acid sequence of SEQ ID NO: 210 to 236 as the germline CDR sequence for kappa light chain CDR-L3; and
2) using a lambda light chain for the germline CDR sequence used for the designing of the light chain CDR and using an amino acid sequence of SEQ ID NO: 237 to 252 as the germline CDR sequence for lambda light chain CDR-L3.

In still another emdbodiment, the present invention provides a method for constructing an antibody library, wherein an utilization frequency of each germline CDR sequence simulates an utilization frequency of each germline CDR sequence in natural human antibodies, which is obtained through the analysis of antibody sequence databases.

In accordance with another aspect of the present inveniton, there is provided an antibody library constructed by the method for constructing an antibody library.

In one specific embodiment, the present inveniton provides an antibody library, wherein the antibody library corresponds to at least one of 1) to 9) below
1) using an amino acid sequence of SEQ ID NO: 54 to SEQ ID NO: 84 as the germline CDR sequence for the heavy chain CDR-H1;
2) using an amino acid sequence of SEQ ID NO: 85 to SEQ ID NO: 121 as the germline CDR sequence for the heavy chain CDR-H2;
3) a form in which the heavy chain CDR-H3 contains 9 to 20 amino acids and the frequency for each length and the utilization frequency of each of amino acid at each position simulate the frequency for each length and the utilization frequency of each of amino acid at each position of CDR-H3 of the natural human antibody sequence;
4) using a kappa light chain for the germline CDR sequence used for the designing of the light chain CDR and using an amino acid sequence of SEQ ID NO: 122 to 145 as the germline CDR sequence for kappa light chain CDR-L1;
5) using a kappa light chain for the germline CDR sequence used for the designing of the light chain CDR and using an amino acid sequence of SEQ ID NO: 146 to 165 as the germline CDR sequence for kappa light chain CDR-L2;
6) using a kappa light chain for the germline CDR sequence used for the designing of the light chain CDR and using an amino acid sequence of SEQ ID NO: 210 to 236 as the germline CDR sequence for kappa light chain CDR-L3;
7) using a lambda light chain for the germline CDR sequence used for the designing of the light chain CDR and using an amino acid sequence of SEQ ID NO: 166 to 189 as the germline CDR sequence for lambda light chain CDR-L1;
8) using a lambda light chain for the germline CDR sequence used for the designing of the light chain CDR and using an amino acid sequence of SEQ ID NO: 190 to 209 as the germline CDR sequence for lambda light chain CDR-L2; and
9) using a lambda light chain for the germline CDR sequence used for the designing of the light chain CDR and using an amino acid sequence of SEQ ID NO: 237 to 252 as the germline CDR sequence for lambda light chain CDR-L3.

In still another specific embodiment, the present invention provides an antibody library, wherein in 1) to 2) and 4) to 9) above, the utilization frequencey of each germline CDR sequence simultates the utiliztion frequency of each germline CDR sequence in natural human antibodies, which is obtained through the analysis of antibody sequence databases.

The terms used in describing the present invention are defined below.

### A. Definitions

The "phage display" is a technique in which a gene of an external protein is fused to a gene of one of surface proteins of engineered genes of M13 bacteriophage, and the external protein is fused to a surface protein of a produced phage to be displayed on a surface of the phage. In cases where a protein is phage displayed, an external gene is often fused at the 5' of gIII gene.

The term "antibody" refers to a protein specifically binding to a target antigen, and encompasses both of a polyclonal antibody and a monoclonal antibody. In addition, the term is intended to encompass any forms produced by genetic engineering, such as chimeric antibodies (e.g., humanized murine antibodies) and heterogeneous antibodies (e.g., bispecific antibodies). Especially, the antibody may be, but is not limited to, a heterotetramer consisting of two light chains and two heavy chains, while each of the chains may include a variable domain having a variable amino acid sequence and a constant domain having a constant amino acid sequence.

As used herein, the antibody includes IgA, IgD, IgE, IgM, and IgG, and the subtypes of IgG include IgG1, IgG2, IgG3, and IgG4, and may include an antibody fragment. The term "antibody fragment" refers to a fragment having an antigen-binding function, and is intended to include an Fc fragment, Fab, Fab', F(ab')₂, scFv, a single variable domain antibody, Fv, and the like, while including an antigen-binding form of the antibody. The "Fc fragment" refers to an end region of an antibody, the end region being capable of binding with a cell surface receptor, such as an Fc receptor, and is composed of second or third constant domains of two heavy chains. The Fab has a structure possessing light chain and heavy chain variable regions, a light chain constant region, and a heavy chain first constant domain (CH1), and has one antigen-binding site. The Fab' is different from Fab in that the former has a hinge region including one or more cysteine residues at the C-terminus of the heavy chain CH1 domain. The F(ab')₂ antibody is generated through a disulfide bond formed between the cysteine residues in the hinge region of Fab'. The Fv (variable fragment) refers to a minimal antibody fragment having only a heavy chain variable region and a light chain variable region. The disulfide-stabilized variable fragment (dsFv) has a structure in which a heavy chain variable region and a light chain variable region are linked to each other by a disulfide bond, while the single chain variable fragment (scFV) generally has a structure in which a heavy chain variable region (VH) and a light chain variable region (VL) are covalently linked to each other by a peptide linker. The single variable domain antibody refers to an antibody fragment composed of only one heavy chain or light chain variable domain.

The antibody of the present invention includes a recombinant single chain Fv fragment (scFv), and includes a bivalent or bispecific molecule, diabody, triabody, and tetrabody, without limitation.

Three "complementarity determining regions (CDRs)" are present at each of the light chain and heavy chain variable domains, in which especially highly variable amino acid sequences in the variable domains are found, while antibodies specific to various antigens may be found due to their high variability. Three heavy chain complementarity determining regions sequentially from the amino terminus to the carboxyl terminus are called CDR-H1, CDR-H2, and CDR-H3, while three light chain complementarity determining regions sequentially from the amino terminus to the carboxyl terminus are called CDR-L1, CDR-L2, and CDR-L3. In one antibody, these six complementarity determining regions are assembled to form an antigen-binding site.

The term "framework region" refers to a region other than the complementarity determining region in the variable domain sequence, and refers to a region which has lower sequence variability and diversity compared with the complementarity determining region and is not in general involved in an antigen-antibody response.

The term "immunoglobulin" refers to a concept that encompasses an antibody and an antibody-like molecule having the same structural characteristics as an antibody and having no antigen specificity.

The "germline immunoglobulin gene" is an antibody gene that is present in animal germ cells and has not undergone the recombination of an immunoglobulin gene or the somatic hypermutation after differentiation into B cells. The number of germline immunoglobulin genes varies depending on the species of animals, but is generally tens to hundreds.

The term "mature antibody" refers to an antibody protein which is expressed from an antibody gene prepared by the recombination of germline immunoglobulin genes or the somatic hypermutation during B cell differentiation.

The term "single chain fragment antibody (scFv)" refers to a protein in which light chain and heavy chain variable domains of an antibody are linked to each other via a linker including a peptide chain having approximately 15 connected amino acids. The scFv protein may have an order of light chain variable domain - linker - heavy chain variable domain, or an order of heavy chain variable domain - linker - light chain variable domain, while having the same or similar antigen specificity compared with its original antibody. The linker is a hydrophilic and flexible peptide chain having glycine and serine, while a sequence of 15 amino acids of "(Gly-Gly-Gly-Gly-Ser)₃" or a similar sequence may be often used.

The term "antibody library" refers to a collection of various antibody genes having different sequences. Very high diversity is required to isolate an antibody specific to any antigen from the antibody library, while a library composed of 10⁹ to 10¹¹ different antibody clones is generally constructed and utilized. The antibody genes constituting the antibody library are cloned to a phagemid vector, and then transformed into *E*. *coli.*

The term "phagemid" vector refers to a plasmid DNA having a phage origin of replication, while usually having an antibiotic-resistant gene as a selection marker. The phagemid vector used in the phage display includes gIII gene of M13 phage or a part thereof, and a library gene is ligated to the 5' end of the gIII gene to be expressed as a fusion protein in *E. coli.*

The term "helper phage" refers to a phage that provides necessary genetic information to allow the phagemid to be assembled into a phage particle. Since only gIII or a part thereof of phage genes exists in phagemid, *E. coli* transformed by phagemid is infected with the helper phage to supply remainder phage genes. The types of helper phage include M13K07 or VCSM13, while most of the helper phages contain antibiotic-resistant genes, such as kanamycin, to allow the selection of *E. coli* infected with the helper phage. In addition, the packaging signal is defective in the helper phage, and thus the phagemid gene, rather than the helper phage gene, is selectively assembled into a phage particle.

The term "panning" refers to a process of selectively amplifying only those clones that bind to a specific molecule from a library of proteins, such as antibodies, displayed on a phage surface. The procedure is that a phage library is added to a target molecule immobilized on the surface to induce binding, unbound phage clones are removed by washing, only bound phage clones are eluted and again infect the *E. Coli* host, and target-bound phage clones are amplified using helper phages. In most cases, this process is repeated three to four times or more to maximize the percentage of bound clones.

### B. Library design, construction, and validation

As an aspect for achieving the purpose, the present invention provides a method for preparing an antibody library, the method comprising: individually designing complementarity determining region (CDR) sequences of antibodies; and synthesizing antibodies comprising the designed complementarity determining region sequences to construct a library.

A human antibody library is constructed using the method described in the present invention, and a human antibody to any antigen can be obtained therefrom. Antibody libraries may be constructed by a method of obtaining diversity from B cells contained in the bone marrow, spleen, blood, or the like, or by a method of obtaining diversity through artificial design and synthesis. The present invention provides the construction and validation of a synthetic human antibody library.

The phage-display antibody library is constructed in the form of a Fab or scFv fragment, which is a part of an immunoglobulin molecule. Since these fragments are smaller than 150 kDa immunoglobulin, the fragments can be engineered efficiency and have the same antigen selectivity as immunoglobulin molecules. In the present invention, a library using an scFv fragment having a size of 25 kDa was constructed. Specifically, the library has a single polypeptide chain in which a VH domain and a VL domain of an immunoglobulin are linked via a chain consisting of 15 amino acids, that is, (Gly-Gly-Gly-Gly-Ser)₃.

The design of the library sequence needs to be preceded in order to construct a synthetic library. Unlike B cell-derived antibody libraries, i.e. natural antibody libraries with relatively high framework diversity, the synthetic antibody library is constructed based on a single or limited number of framework sequences.

The antibody library constructed in a specific embodiment of the present invention has two frameworks. Specifically, the library was constructed such that all the clones constituting the library have, as frameworks, scFv having a human immunoglobulin VH3-23 gene and a human immunoglobulin VK3-A27 gene linked via a linker, or scFv having a human immunoglobulin VH3-23 gene and a human immunoglobulin VL1g gene linked via a linker, and artificial diversity was introduced to complementarity determining regions of the framework. That is, various complementarity determining region (CDR) sequences were grafted into the framework of the library to construct an scFv antibody library.

Specifically, heavy chain complementarity determining region 1 (CDR-H1), heavy chain complementarity determining region 2 (CDR-H2), heavy chain complementarity determining region 3 (CDR-H3), light chain complementarity determining region 1 (CDR-L1), light chain complementarity determining region 2 (CDR-L2), and light chain complementarity determining region 3 (CDR-L3), which constitute the complementarity determining regions of the antibodies included in the antibody library, may have diversity.

Especially, in the individual designing of the complementarity determining region sequences, for CDR-H1, CDR-H2, CDR-L1, or CDR-L2, the sequences therefor are designed by simulating i) an utilization frequency of each germline immunoglobulin gene, ii) a frequency of mutation into each of 20 amino acids by somatic hypermutations at each amino acid, iii) a length distribution frequency of sequences comprising each complementarity determining region, or iv) a frequency of each amino acid at each position calculated by analyzing a combination thereof, of the complementarity determining regions of actual human-derived mature antibodies.

Meanwhile, in the individual designing of the complementarity determining region sequences, for CDR-L3,
a) 7 or 8 amino acid sequences from a N-terminus of the complementarity determining region are designed by simulating i) an utilization frequency of each germline immunoglobulin gene, ii) a frequency of mutation into each of 20 amino acids by somatic hypermutations at each amino acid position, iii) a length distribution frequency of sequences comprising CDR-L3, or iv) a frequency of each amino acid at each position calculated by analyzing a combination thereof, of the complementarity determining region of actual human-derived mature antibodies, and
b) 2 or 3 amino acid sequences from a C-terminus of the complementarity determining region are designed by analyzing and calculating a frequency of each amino acid at each position in the complementarity determining region of actual human-derived mature antibodies, then simulating sequences that reflect the calculated frequencies; and
wherein the CDR-L3 contains 9 to 11 amino acids and the analysis of the frequencies is conducted according to each length, the CDR-L3 sequences being designed based on an analysis result of complementarity determining region CDR-L3 of human-derived mature antibodies, which have the same amino acid lengths as CDR-L3 to be designed.

Furthermore, in the individual designing of the complementarity determining region sequences, for CDR-H3,
a) each sequence therefor excluding three amino acids from a C-terminus of the complementarity determining region is designed by using a frequency of each amino acid at each position in the complementarity determining region of actual human-derived mature antibodies, and
b) a 3 amino acid sequence from the C-terminus of the complementarity determining region is designed by analyzing and calculating frequencies of the corresponding 3 amino acid sequences in the complementarity determining region of actual human-derived mature antibodies, then simulating sequences that reflect the calculated frequencies; and
wherein the CDR-H3 contains 9 to 20 amino acids and the analysis of the frequencies is conducted according to each length, the CDR-H3 sequences being designed based on an analysis result of complementarity determining region CDR-H3 of human-derived mature antibodies, which have the same amino acid length as CDR-H3 to be designed.

As for the germline CDR sequences used in the design of the library heavy chain CDR sequences of the present invention, an amino acid sequence of SEQ ID NO: 54 to SEQ ID NO: 84 may be used as the germline CDR sequence for CDR-H1, while an amino acid sequence of SEQ ID NO: 85 to SEQ ID NO: 121 may be used as the germline CDR sequence for CDR-H2. Meanwhile, in the design of CDR-H3, while CDR-H3 sequences is prepared to have different lengths of 9 to 20 amino acids, without using germline CDR sequences, the CDR-H3 sequences may be designed such that the frequency for each length and the utilization frequency of each of amino acid at each position simulate the frequency for each length and the utilization frequency of each of amino acid at each position of CDR-H3 of the natural human antibody sequence.

In addition, the germline CDR sequences used in the design of the library light chain CDR sequences of the present invention may have a lambda light chain or kappa light chain. The germline CDR sequence for CDR-L1 of the kappa light chain may have an amino acid sequence of SEQ ID NO: 122 to SEQ ID NO: 145, while the germline CDR sequence for CDR-L2 of the kappa light chain may have an amino acid sequence of SEQ ID NO: 146 to SEQ ID NO: 165. Meanwhile, the germline CDR sequence for CDR-L3 of the kappa light chain may have an amino acid sequence of SEQ ID NO: 210 to SEQ ID NO: 236.

Meanwhile, the germline CDR sequence for CDR-L1 of the lambda light chain of the present invention may have an amino acid sequence of SEQ ID NO: 166 to 189, while the germline CDR sequence for CDR-L2 of the lambda light chain may have an amino acid sequence of SEQ ID NO: 190 to SEQ ID NO: 209. Further, the germline CDR sequence for CDR-L3 of the lambda light chain may have an amino acid sequence of SEQ ID NO: 237 to SEQ ID NO: 252.

The method for preparing or constructing an antibody library, especially, the method for desiging CDR-H1, CDR-H2, CDR-L1, and CDR-L2, which are included in the antibody library of the present invention, may correspond to at least one of 1) to 6) below:
1) using an amino acid sequence of SEQ ID NO: 54 to SEQ ID NO: 84 as the germline CDR sequence for the heavy chain CDR-H1;
2) using an amino acid sequence of SEQ ID NO: 85 to SEQ ID NO: 121 as the germline CDR sequence for the heavy chain CDR-H2;
3) using a kappa light chain for the germline CDR sequence used for the designing of the light chain CDR and using an amino acid sequence of SEQ ID NO: 122 to 145 as the germline CDR sequence for kappa light chain CDR-L1;
4) using a kappa light chain for the germline CDR sequence used for the designing of the light chain CDR and using an amino acid sequence of SEQ ID NO: 146 to 165 as the germline CDR sequence for kappa light chain CDR-L2;
5) using a lambda light chain for the germline CDR sequence used for the designing of the light chain CDR and using an amino acid sequence of SEQ ID NO: 166 to 189 as the germline CDR sequence for lambda light chain CDR-L1; and
6) using a lambda light chain for the germline CDR sequence used for the designing of the light chain CDR and using an amino acid sequence of SEQ ID NO: 190 to 209 as the germline CDR sequence for lambda light chain CDR-L2.

In addition, the method for preparing or constructing an antibody library, especially, the method for desiging CDR-L3, which is included in the antibody library of the present invention, may correspond to at least one of 1) to 2) below:
1) using a kappa light chain for the germline CDR sequence used for the designing of the light chain CDR and using an amino acid sequence of SEQ ID NO: 210 to 236 as the germline CDR sequence for kappa light chain CDR-L3; and
2) using a lambda light chain for the germline CDR sequence used for the designing of the light chain CDR and using an amino acid sequence of SEQ ID NO: 237 to 252 as the germline CDR sequence for lambda light chain CDR-L3.

The utilization frequency of each germline CDR sequence may simulate the utilization frequency of each germline CDR sequence in a natural human antibody, which is obtained through the analysis of antibody sequence database.

In a specific embodiment of the present invention, as for the germline CDR sequences used in the design of the library heavy chain CDR sequences of the present invention, an amino acid sequence of SEQ ID NO: 54 to SEQ ID NO: 84 may be used as the germline CDR sequence for CDR-H1, while an amino acid sequence of SEQ ID NO: 85 to SEQ ID NO: 121 may be used as the germline CDR sequence for CDR-H2. In the design of CDR-H3, while CDR-H3 sequences is prepared to have different lengths of 9 to 20 amino acids, the CDR-H3 sequences may be designed such that the frequency for each length and the utilization frequency of each of amino acid at each position simulate the frequency for each length and the utilization frequency of each of amino acid at each position of CDR-H3 of the natural human antibody sequences.

In addition, as for the germline CDR sequences used in the design of the library light chain CDR sequences of the present invention, in the lambda light chain or kappa light chain, the germline CDR sequence for CDR-L1 of the kappa light chain may have an amino acid sequence of SEQ ID NO: 122 to SEQ ID NO: 145, while the germline CDR sequence for CDR-L2 of the kappa light chain may have an amino acid sequence of SEQ ID NO: 146 to SEQ ID NO: 165. Meanwhile, the germline CDR sequence for CDR-L3 of the kappa light chain may have an amino acid sequence of SEQ ID NO: 210 to SEQ ID NO: 236.

Meanwhile, the germline CDR sequence for CDR-L1 of the lambda light chain of the present invention may have an amino acid sequence of SEQ ID NO: 166 to 189, while the germline CDR sequence for CDR-L2 of the lambda light chain may have an amino acid sequence of SEQ ID NO: 190 to SEQ ID NO: 209. Meanwhile, the germline CDR sequence for CDR-L3 of the lambda light chain may have an amino acid sequence of SEQ ID NO: 237 to SEQ ID NO: 252.

According to the method for preparing or constructing an antibody library, in the individual designing of the complementarity determining region sequences, when light chain complementarity determinig regions are designed, each CDR may be designed for the kappa light chain and the lambda light chain. When each light chain complementarity determinig region is designed for the kappa light chain and the lambda light chain, respectively, kappa light chain CDR may be assembled by linkage with a kappa light chain framework region of VK3-A27, while lambda light chain CDR may be assembled by linkage with a lambda light chain framework region of VL1g.

As used herein, the term "simulation" refers to the designing of a sequence by reflecting the expression frequency or modification frequency of amino acid sequences or the like to perform random simulation, and encompasses the meaning of simulating the expression frequency or modification frequency of amino acid sequences especially in human-derived mature antibodies.

In a specific embodiment of the present invention, as for complementarity determining region diversity, CDR sequences were designed to have similar sequence diversity to human antibody CDRs by analyzing and simulating characteristics of CDR sequences of known human antibodies. Specifically, first, from the (IMGT) database (http://imgt.org), 8,846 human immunoglobulin heavy chain variable region (VH) sequences, 3,110 kappa light chain variable region (Vκ) sequences, and 2,440 lambda light chain variable region (Vλ) sequences were respectively downloaded to extract CDR sequences. Then, CDR sequences of human antibody germline immunoglobulin genes from V-base (http://www2.mrc-lmb.cam.ac.uk/vbase/alignments2.php) were compared and analyzed with mature CDR sequences extracted from the IMGT database. As a result, (i) the germline CDR sequence, which is the closest to each mature CDR sequence, was found, and the position, kind, and frequency of mutations occurring in each mature CDR were determined, and (ii) the utilization frequency of each germline CDR in the mature human antibodies was calculated and used in designing CDR sequences.

First, for heavy chain and light chain CDR1s and CDR2s with only somatic hypermutation without recombination, CDR sequences were designed to have similar sequences and germline CDR sequences of the human-derived mature antibodies by introducing virtual mutations into the human germline CDR sequence, through simulation using a computer. 1,500 simulated sequences for each CDR were designed.

Next, for light chain CDR3 (CDR-L3) with recombination and somatic hypermutation, in cases of the kappa light chain, a total of 1,500 sequences composed of nine or ten amino acids were designed by performing the same work as in CDR1 and CDR2 on first seven amino acids and allowing the last two- or three-amino acid sequence to simulate the frequency of each amino acid in the corresponding portions of kappa light chain CDR3 of the mature human antibodies. In cases of the lambda light chain, a total of 1,500 sequences composed of nine, ten, or eleven amino acids were designed by performing the same work as in CDR1 and CDR2 on first seven or eight amino acids and allowing the last two- or three-amino acid sequence to simulate the frequencies of amino acids in the corresponding positions of lambda light chain CDR3 of the mature human antibodies.

Last, in heavy chain CDR3 with recombination and somatic hypermutation, the identification of the germline sequence is often difficult due to the VDJ recombination and such a mechanism as junctional flexibility, P-addition, or N-addition, and thus, the same analysis as in the other CDRs is restricted. Therefore, CDR-H3 sequences of the mature human antibodies having 9 to 20 amino acid lengths were analyzed for the utilization frequency of each of amino acids at each position of each length, and these data were used for simulation. However, most of the last three-amino acid sequences are derived from J gene, and thus, a maximum of eight 3-amino acid sequences most frequently used for each CDR-H3 length in the mature human antibodies designed considering the utilization frequency in the mature antibodies (FIG. 1).

Especially, in the designing of heavy chain CDR-H3, the frequency for each length and the utilization frequency of each of amino acid at each position of CDR-H3 of the natural human antibodies can be obtaind through the analysis of antibody sequence database, and representatively, antibody sequences collected in the IMGT database (http://www.imgt.org) may be analyzed.

Meanwhile, the method for preparing or constructing an antibody library of the present invention may comprise, after the designing of the complementarity determining region amino acid sequences, excluding sequences having the possibility of occurrence of N-glycosylation, isomerization, deamidation, cleavage, and oxidation from the designed sequences.

In a specific embodiment of the present invention, in the designing of CDR sequences, sequences including the following post-translational modification sequences were excluded. The post-translational modifications of proteins may affect the functions and physicochemical properties of the proteins, and thus it is advantageous to exclude such sequences as much as possible in the design of the antibody library. The post-translatonal modification sequences excluded an N-glycosylation sequence, i.e., Asp-Xaa-Ser/Thr (Asp: aspartic acid, Xaa: any of 19 kinds of amino acids other than proline, Ser/Thr: serine or threonine), an isomerization sequence, a deamidation sequence, a cleavage sequence, and an oxidation sequence.

Meanwhile, the method for preparing or constructing an antibody library of the present invention may include, after the designing of the complementarity determining region amino acid sequences, reverse-translating the designed sequences into polynucleotide sequences and then designing oligonucleotide sequences, in which framework region sequences of variable regions of a human antibody germline gene flanking the complementarity determining region are linked to the 5' and 3' ends of the reverse-translated polynucleotide.

In one specific embodiment of the present invention, the designed CDR amino acid sequences were reversely translated into nucleotide sequences, and the framework sequences of the antibody variable regions were added at both sides of the CDR sequence, thereby finally designing oligonucleotide sequences composed of 100 nucleotides. A total of such 19,836 sequences were designed, and synthesized in the form of an oligonucleotide mixture through an array synthesis method (LC Sciences, Houston, Texas, USA).

In the method for constructing an antibody library of the present invention, the antibodies may include amino acid sequences encoded by VH3-23, VK3-A27, VL1g, or fragments thereof. VH3-23 may correspond to Genebank accession No. Z12347, VK3-A27 to Genebank accession No. X93639, and VL1g to GenBank accession No. Z73663.

In one specific embodiment, codon-optimized scFv genes were synthesized (Genscript, Piscataway, NJ) for use as frameworks for antibody library construction. These genes are scFv genes in which germline genes VH3-23 and VK3-A27, or VH3-23 and VL1g were connected to each other via a linker of (Gly-Gly-Gly-Gly-Ser)₃, and were cloned to pUC57 vector, and for cloning into the phagemid vector, two SfiI restriction enzyme sites, which are compatible with pComb3X vector, were included. For convenience of library construction, these genes cloned into pUC57 vector were also cloned into pComb3X vector, and then used as a template for PCR. These genes are intended to be used as frameworks, and thus are single sequences without sequence diversity, and the codons were optimized to improve the expression in mammalian cells, while in order to prevent non-specific annealing in the PCR process, the codon-optimized DNA sequences were partially changed without changing the translated amino acid sequences (SEQ ID NO: 19 and SEQ ID NO: 20).

In the method for constructing an antibody library of the present invention, the antibodies may be selected from the group consisting of IgA, IgD, IgE, IgM, IgG, Fc fragments, Fab, Fab', F(ab')₂, scFv, a single variable domain antibody, and Fv.

Especially, in one specific embodiment of the present invention, the scFv antibody library was constructed, and the expression of scFv was confirmed in the constrcted library (FIG. 2). In order to verify the construction of the antibody library, the sequences of scFvs were also analyzed. Random clones were selected from each library and scFv genes were amplified through PCR technique, and then DNA was purified and subjected to sequencing. As a result of sequencing analysis, it was confirmed that CDRs having various designed sequences and lengths were introduced into scFv clones constituting the library (FIG. 3). As a result of detailed sequence analysis, it was confirmed that there were no un-intended post-translational modification sequences except for one case in which the cleavage (asparatic acid-proline) sequence was introduced by a PCR error in one of 18 scFv sequences analyzed. Whereas, as a result of analyzing the percentage of presence of sequences that could induce such post-translational modifications in each CDR of human-derived mature antibodies, the percentage was 5.7% for CDR-H1, 39.7% for CDR-H2, and 34.5% for CDR-H3 in the heavy chain; 12.6% for CDR-L1, 0.6% for CDR-L2, and 15.9% for CDR-L3 in the kappa light chain; and 8.3% for CDR-L1, 6.4% for CDR-L2, and 24.0% for CDR-L3 in the lambda light chain. Therefore, it was confirmed that the antibody library capable of minimizing post-translational modification sequences as designed was constructed.

In accordance with another aspect of the present invention, there is provided an antibody library constructed by the method for constructing an antibody library of the present invention.

In the antibody library of the present invention, an amino acid sequence of SEQ ID NO: 54 to SEQ ID NO: 84 may be used as the germline CDR sequence for CDR-H1, and an amino acid sequence of SEQ ID NO: 85 to SEQ ID NO: 121 may be used as the germline CDR sequence for CDR-H2. Meanwhile, in the design of CDR-H3, while CDR-H3 sequences are prepared to have different lengths of 9 to 20 amino acids, the CDR-H3 sequences may be designed such that the frequency for each length and the utilization frequency of each of amino acid at each position simulate the frequency for each length and the utilization frequency of each of amino acid at each position of CDR-H3 of the natural human antibody sequences.

The antibody library of the present invention may use a lambda light chain or kappa light chain for the germline CDR sequences used in the design of the library light chain CDR sequences. The germline CDR sequence for CDR-L1 of the kappa light chain may be preprared by using an amino acid sequence of SEQ ID NO: 122 to SEQ ID NO: 145, and the germline CDR sequence for CDR-L2 of the kappa light chain may be prepared by using an amino acid sequence of SEQ ID NO: 146 to SEQ ID NO: 165. Meanwhile, the germline CDR sequence for CDR-L3 of the kappa light chain may be prepared by using an amino acid sequence of SEQ ID NO: 210 to SEQ ID NO: 236.

Meanwhile, the germline CDR sequence for CDR-L1 of the lambda light chain of the present invention may be prepared by using an amino acid sequence of SEQ ID NO: 166 to 189, and the germline CDR sequence for CDR-L2 of the lambda light chain may be prepared by using an amino acid sequence of SEQ ID NO: 190 to SEQ ID NO: 209. Meanwhile, the germline CDR sequence for CDR-L3 of the lambda light chain may be prpared by using an amino acid sequence of SEQ ID NO: 237 to SEQ ID NO: 252.

Specifially, the antibody library of the present invention may correspnd to at least one of 1) to 9) below.
1) using an amino acid sequence of SEQ ID NO: 54 to SEQ ID NO: 84 as the germline CDR sequence for the heavy chain CDR-H1;
2) using an amino acid sequence of SEQ ID NO: 85 to SEQ ID NO: 121 as the germline CDR sequence for the heavy chain CDR-H2;
3) a form in which the heavy chain CDR-H3 contains 9 to 20 amino acids and the frequency for each length and the utilization frequency of each of amino acid at each position simulate the frequency for each length and the utilization frequency of each of amino acid at each position of CDR-H3 of the natural human antibody sequences;
4) using a kappa light chain for the germline CDR sequence used for the designing of the light chain CDR and using an amino acid sequence of SEQ ID NO: 122 to 145 as the germline CDR sequence for kappa light chain CDR-L1;
5) using a kappa light chain for the germline CDR sequence used for the designing of the light chain CDR and using an amino acid sequence of SEQ ID NO: 146 to 165 as the germline CDR sequence for kappa light chain CDR-L2;
6) using a kappa light chain for the germline CDR sequence used for the designing of the light chain CDR and using an amino acid sequence of SEQ ID NO: 210 to 236 as the germline CDR sequence for kappa light chain CDR-L3;
7) using a lambda light chain for the germline CDR sequence used for the designing of the light chain CDR and using an amino acid sequence of SEQ ID NO: 166 to 189 as the germline CDR sequence for lambda light chain CDR-L1;
8) using a lambda light chain for the germline CDR sequence used for the designing of the light chain CDR and using an amino acid sequence of SEQ ID NO: 190 to 209 as the germline CDR sequence for lambda light chain CDR-L2; and
9) using a lambda light chain for the germline CDR sequence used for the designing of the light chain CDR and using an amino acid sequence of SEQ ID NO: 237 to 252 as the germline CDR sequence for lambda light chain CDR-L3.

Especialy, the present invention may be an antibody library, wherein in 1) to 2) and 4) to 9) above, the utilization frequencey of each germline CDR sequence simultates the utiliztion frequency of each germline CDR sequence in natural human antibodies, which is obtained through the analysis of antibody sequence database.

In one specific embodiment of the present invention, the transformed *E. coli* library was incubated, and infected with VCSM13 helper phage, to obtain an scFv displayed antibody phage library. Using this library, the functionality of the antibody phage library was validated by a panning experiment of selecting antigen-specific antibodies against lysozyme (Hen egg white lysozyme, HEWL) and AIMP1 (101-192) antigens. As a result, 188 clones were screened by ELISA for HEWL antigens, while 150 ELISA positive clones showing a binding signal by 3-fold or higher compared with the background signal were identified. Among these positive clones, 16 clones were sequenced to find five unique sequences. In addition, 94 clones for AIMP1 (101-192) were screened by ELISA, while 18 ELISA positive clones showing a binding signal by 3-fold or more compared with a background signal were identified. Among these positive clones, five clones were sequenced to find two unique sequences.

The technique of design and construction of the antibody library according to the present invention may be used to construct an improved library having, as frameworks, a plurality of human germline immunoglobulin variable region genes.

That is, the antibody library described in the present invention may be constructed according to a design manner in which one framework for each of heavy chain, kappa light chain, and lambda light chain (VH3-23, VK3-A27, and VL1g, respectively) is used and synthetic CDRs, obtained by introducing mutations to CDR sequences derived from various different germline immunoglobulin genes, are inserted thereinto, and also a library constructed to have a similar connecting combination between framework and CDRs to natural human derived antibodies may be obtained by, on the basis of framework genes of a plurality of various germline immunoglobulin variable regions, improved library design of introducing, into the framework genes, synthetic CDRs obtained by introducing mutations into CDR sequences derived from germline immunoglobulin genes of the same gene group as each corresponding framework.

In addition, in order to solve the bias that short CDR sequences are preferred due to the differences in synthesis and amplification efficiency according to the CDR length in the construction of the antibody library, sub-libraries may be constructed for each different CDR lengths, and then mixed at a similar ratio to the distribution of CDR lengths found in natural human antibodies, thereby constructing a final library.

In accordance to still another aspect, the present invention provides antibodies produced from the antibody library constructed by the method for constructing an antibody library of the present invention.

In the present invention, the antibody, antibody library, and the like are described as above.

### Mode for Carrying Out the Invention

Hereinafter, the present invention will be described in detail. However, these examples are given for specifically illustrating the present invention, and the scope of the present invention is not limited thereto.

### Example 1: Construction of antibody library

A synthetic human antibody library was intended to be constructed in the present invention. Especially, an antibody library using an scFV fragment having a size of 25 kDa was constructed. In a specific embodiment, the antibody of the present invention was produced by linking VH domain and VL domain of the immunoglobulin via a chain composed of 15 amino acids, that is, (Gly-Gly-Gly-Gly-Ser)₃.

Since the design of library sequences needs to be preceded in order to construct a synthetic library, the present inventors designed the sequences as follows.

### 1-1. Design of antibody library framework sequences

Unlike a B cell-derived antibody library or a natural antibody library with relatively high framework diversity, a synthetic antibody library is constructed based on a single or limited number of framework sequences. In an exemplified embodiment of the present invention, the antibody library was constructed using two antibody frameworks.

Specifically, the library was constructed such that all the clones constituting the antibody library of the present invention have, as frameworks, scFv having VH3-23 gene and VK3-A27 gene of the human immunoglobulin linked to each other via a linker, or scFv having VH3-23 gene and VL1g gene of the human immunoglobulin linked to each other via a linker, while an artificial diversity was introduced to complementarity determining regions of the frameworks. The frameworks used therefor are shown as in Table 1 below.

**[Table 1]**

| Framework form | Sequence |
|---|---|
| (VH3-23)-linker-(Vk3-A27) | 5'- gaagtgcagctgctggaaagtggaggtggactggtgcagcctggcggcagcctg cgcctgagctgtgccgccagcggattcaccttcagcNNNNNNNNNNNNNNNtgg gttcgccaagcacctggcaaaggcctggaatgggtgNNNNNNNNNNNNNNNcgc tttaccatcagccgcgataacagcaaaaacaccctgtatctgcagatgaacagc ctgcgcgccgaggacaccgcagtctactactgtNNNNNNNNNNNNNNNtgggga caaggtactctggtgaccgtgagcagcggtggaggaggtagcggaggtggtgga tctggaggtggaggtagtgaaatcgtgctgacccagagccctggcaccctgagc ctgagccctggcgaacgcgcaacactgtcatgcNNNNNNNNNNNNNNNtggtat cagcagaaaccaggtcaggctccacgtctgctgatctatNNNNNNNNNNNNNNN ggcatccctgatcgcttctcaggatctggaagcggtaccgattttaccctgacc atcagccgcctggaacctgaggactttgccgtgtattattgtNNNNNNNNNNNN NNNttcggtcagggcactaaagtggaaatcaaa-3' (SEQ ID NO: 50) |
| (VH3-23)-linker-(Vk3-A27) | N'- EVQLLESGGGLVQPGGSLRLSCAASGFTFSXXXXXWVRQAPGKGLEWVXXXXXR FTISRDNSKNTLYLQMNSLRAEDTAVYYCAKXXXXXWGQGTLVTVSSGGGGSGG GGSGGGGSEIVLTQSPGTLSLSPGERATLSCXXXXXWYQQKPGQAPRLLIYXXX XXGIPDRFSGSGSGTDFTLTISRLEPEDFAVYYCXXXXXFGQGTKVEIK-C' (SEQ ID NO: 51) |
| (VH3-23)-linker-(Vl1g) | 5'- gaagtgcagctgctggaaagtggaggtggactggtgcagcctggcggcagcctg cgcctgagctgtgccgccagcggattcaccttcagcNNNNNNNNNNNNNNNtgg gttcgccaagcacctggcaaaggcctggaatgggtgNNNNNNNNNNNNNNNcgc tttaccatcagccgcgataacagcaaaaacaccctgtatctgcagatgaacagc ctgcgcgccgaggacaccgcagtctactactgtNNNNNNNNNNNNNNNtgggga caaggtactctggtgaccgtgagcagcggtggaggaggtagcggaggtggtgga tctggaggtggaggtagtcagagcgtgctgacccagcctcctagcgcctccggt acaccaggacagcgcgtgactattagctgtNNNNNNNNNNNNNNNtggtaccag caactgcctggaactgcacctaagctgctgatctatNNNNNNNNNNNNNNNggc gtgcctgatcgctttagcggtagcaaatcaggcaccagcgccagcctggccatc agcggccttcgctccgaagatgaagccgattattattgtNNNNNNNNNNNNNNN tttggtggcggtaccaagctgaccgtgctg-3' (SEQ ID NO: 52) |
| (VH3-23)-linker-(Vl1g) | N'- EVQLLESGGGLVQPGGSLRLSCAASGFTFSXXXXXWVRQAPGKGLEWVXXXXXR FTISRDNSKNTLYLQMNSLRAEDTAVYYCAKXXXXXWGQGTLVTVSSGGGGSGG GGSGGGGSQSVLTQPPSASGTPGQRVTISCXXXXXWYQQLPGTAPKLLIYXXXX XGVPDRFSGSKSGTSASLAISGLRSEDEADYYCXXXXXFGGGTKLTVL-C' (SEQ ID NO: 53) |

In the above Table, N represents any nucleotide in the nucleotide sequences, while X represents any amino acid in the amino acid sequences. The underlined parts represent CDRs, and the parts marked in bold represent linkers.

### 1-2. Design of complementarity determining region (CDR) sequences of antibody library

Various complementarity determining region (CDR) sequences were grafted into the frameworks of the antibody library to construct an scFv antibody library. As for CDR diversity, CDR sequences were designed to have similar sequence diversity to human antibody CDRs by analyzing and simulating characteristics of CDR sequences of known human antibodies. A specific method is as follows.
- From the (IMGT) database (http://imgt.org), 8,846 human immunoglobulin heavy chain variable region (V_{H}) sequences, 3,110 kappa light chain variable region (V_{κ}) sequences, and 2,440 lambda light chain variable region (V_{λ}) sequences were downloaded to extract CDR sequences.
   The downloaded variable region sequences are different sequences having three CDRs at each variable region. In order to extract CDRs, the rules found in http://www.bioinf.org.uk/abs/index.html#cdrid were used.
- CDR sequences of human antibody germline immunoglobulin genes from V-base (http://www2.mrc-lmb.cam.ac.uk/vbase/alignments2.php) were compared and analyzed with mature CDR sequences extracted from the IMGT database.

As a result, (i) the germline CDR sequence, which is the closest to each mature CDR sequence, was found, and the position, kind, and frequency of mutations occurring in each mature CDR sequence were investigated, and (ii) the utilization frequency of each germline CDR in the mature human antibodies was investigated.

As for the germline CDR sequences used in the design of the library heavy chain CDR sequences of the present invention, an amino acid sequence of SEQ ID NO: 54 to SEQ ID NO: 84 was used as the germline CDR sequence for CDR-H1, while an amino acid sequence of SEQ ID NO: 85 to SEQ ID NO: 121 was used as the germline CDR sequence for CDR-H2. Meanwhile, in the design of CDR-H3, while CDR-H3 sequences were prepared to have different lengths of 9 to 20 amino acids, without using germline CDR sequences, the CDR-H3 sequences were designed such that the frequency for each length and the utilization frequency of each of amino acid at each position simulate the frequency for each length and the utilization frequency of each of amino acid at each position of CDR-H3 of the natural human antibody sequences.

Meanwhile, the germline CDR sequences used in the design of the library light chain CDR sequences of the present invention may have a lambda light chain or kappa light chain. The germline CDR sequence for CDR-L1 of the kappa light chain has an amino acid sequence of SEQ ID NO: 122 to SEQ ID NO: 145, while the germline CDR sequence for CDR-L2 of the kappa light chain has an amino acid sequence of SEQ ID NO: 146 to SEQ ID NO: 165. Meanwhile, the germline CDR sequence for CDR-L3 of the kappa light chain has an amino acid sequence of SEQ ID NO: 210 to SEQ ID NO: 236.

Meanwhile, the germline CDR sequence for CDR-L1 of the lambda light chain of the present invention has an amino acid sequence of SEQ ID NO: 166 to 189, while the germline CDR sequence for CDR-L2 of the lambda light chain has an amino acid sequence of SEQ ID NO: 190 to SEQ ID NO: 209. Meanwhile, the germline CDR sequence for CDR-L3 of the lambda light chain has an amino acid sequence of SEQ ID NO: 237 to SEQ ID NO: 252.
- For heavy chain and light chain CDR1s and CDR2s with only somatic hypermutation without recombination, the following work was conducted. That is, CDR sequences were designed to have similar sequences and germline CDR sequence utilization frequencies to the CDR sequences of the human-derived mature antibodies by introducing virtual mutations into the human germline CDR sequence, through simulation using a computer. 1,500 simulated sequences for each CDR were designed.
- For light chain CDR3 (CDR-L3) with recombination and somatic hypermutation, in cases of the kappa light chain, a total of 1,500 sequences composed of nine or ten amino acids were designed by performing the same work as in CDR1 and CDR2 on first seven amino acids and allowing the last two- or three-amino acid sequence to simulate the frequency of each amino acid in the corresponding positions of kappa light chain CDR3 of the mature human antibodies. In cases of the lambda light chain, a total of 1,500 sequences composed of nine, ten, or eleven amino acids were designed by performing the same work as in CDR1 and CDR2 on first seven or eight amino acids and allowing the last two- or three-amino acid sequence to simulate the frequencies of amino acids in the corresponding positions of lambda light chain CDR3 of the mature human antibodies.
- Specifically, the amino acid frequencies of each position of two- to three-amino acid sequences from the C-terminus of the CDR-L3 are shown in Table 2 below.

**[Table 2]**

| Amino acid | CDR-L3 (%) | | | | | | | | | | | | |
|---|---|---|---|---|---|---|---|---|---|---|---|---|---|
| | Kappa CDR-L3 length of 9 | | Kappa CDR-L3 length of 10 | | | Lambda CDR-L3 length of 9 | | Lambda CDR-L3 length of 10 | | | Lambda CDR-L3 length of 11 | | |
| | 8th | 9th | 8th | 9th | 10th | 8th | 9th | 8th | 9th | 10th | 9th | 10th | 11th |
| A | 0.4 | 2.5 | 1.2 | 0.0 | 0.4 | 12.8 | 0.6 | 3.7 | 2.5 | 1.0 | 13.2 | 1.2 | 0.6 |
| C | 0.7 | 0.0 | 0.2 | 1.8 | 0.0 | 0.0 | 0.0 | 0.0 | 1.2 | 0.0 | 0.0 | 0.3 | 0.0 |
| D | 0.4 | 0.1 | 0.2 | 0.8 | 0.0 | 0.6 | 0.0 | 2.9 | 0.2 | 0.0 | 0.8 | 0.5 | 0.0 |
| E | 0.2 | 0.0 | 0.2 | 0.4 | 0.0 | 1.1 | 0.0 | 0.2 | 1.0 | 0.0 | 0.5 | 1.9 | 0.0 |
| F | 7.2 | 0.0 | 0.4 | 10.1 | 0.0 | 0.0 | 0.0 | 1.2 | 3.4 | 0.0 | 2.9 | 0.8 | 0.0 |
| G | 1.5 | 0.3 | 2.8 | 1.8 | 0.2 | 5.6 | 0.0 | 2.9 | 4.9 | 0.0 | 32.9 | 5.7 | 0.0 |
| H | 2.1 | 0.1 | 0.4 | 1.4 | 0.2 | 0.6 | 0.0 | 4.7 | 0.7 | 0.0 | 26.5 | 0.2 | 0.0 |
| I | 6.2 | 0.2 | 0.6 | 9.3 | 0.4 | 1.7 | 6.7 | 2.7 | 0.2 | 6.9 | 0.2 | 0.0 | 2.8 |
| K | 0.2 | 0.0 | 0.2 | 0.4 | 0.0 | 1.7 | 0.0 | 0.7 | 0.0 | 0.0 | 0.1 | 0.3 | 0.0 |
| L | 22.7 | 0.3 | 7.5 | 14.1 | 0.2 | 2.8 | 3.9 | 3.2 | 16.4 | 3.9 | 6.8 | 6.5 | 5.2 |
| M | 0.1 | 0.1 | 2.8 | 0.0 | 0.2 | 0.6 | 0.0 | 0.0 | 0.5 | 0.0 | 0.0 | 0.4 | 0.0 |
| N | 1.7 | 0.1 | 0.0 | 1.2 | 0.2 | 0.6 | 0.0 | 14.7 | 0.0 | 0.0 | 0.3 | 0.5 | 0.0 |
| P | 7.6 | 0.3 | 59.4 | 0.0 | 0.0 | 1.1 | 0.0 | 2.2 | 0.5 | 0.0 | 2.5 | 8.9 | 0.0 |
| Q | 4.9 | 0.0 | 2.0 | 0.0 | 0.0 | 6.7 | 0.0 | 0.7 | 1.2 | 0.0 | 1.3 | 1.7 | 0.0 |
| R | 16.2 | 0.1 | 14.7 | 1.6 | 0.0 | 4.5 | 0.0 | 1.5 | 7.8 | 0.0 | 1.4 | 7.0 | 0.0 |
| S | 0.8 | 2.8 | 4.4 | 1.2 | 4.0 | 0.6 | 0.0 | 8.1 | 0.7 | 0.0 | 2.9 | 2.7 | 0.0 |
| T | 0.4 | 92.8 | 2.0 | 0.6 | 94.1 | 1.7 | 0.0 | 47.5 | 0.2 | 0.0 | 1.3 | 0.0 | 0.0 |
| V | 1.1 | 0.3 | 0.6 | 3.8 | 0.0 | 42.5 | 88.8 | 0.5 | 25.2 | 88.2 | 2.3 | 29.7 | 91.4 |
| W | 10.9 | 0.0 | 0.2 | 27.5 | 0.0 | 9.5 | 0.0 | 0.0 | 22.8 | 0.0 | 0.1 | 18.4 | 0.0 |
| Y | 14.9 | 0.0 | 0.0 | 23.8 | 0.0 | 5.6 | 0.0 | 2.5 | 10.3 | 0.0 | 4.2 | 13.1 | 0.0 |

Therefore, in cases of CDR-L3, the front seven-amino acid (eight for lambda light chain CDR-L3 having a length of 11 amino acids) sequence is selected based on the germline CDR sequence, and then two or three amino acids were added based on the frequencies in Table 2 above. The reason is that, due to the V-J recombination, the sequence of the C-terminus of CDR-L3 is not often derived from the germline sequence and has high uncertainty.
- In heavy chain CDR3 with recombination and somatic hypermutation, the identification of the germline sequences is difficult due to VDJ recombination and such mechanisms as junctional flexibility, P-addition, or N-addition, and thus, the same analysis as in the other CDRs is not practical.

Therefore, CDR-H3 sequences of the mature human antibodies 9 to 20 amino acid lengths were analyzed for the utilization frequency of each of amino acids at each position of each length, and these data were used for simulation. However, most of the last three-amino acid sequences are derived from J gene, and thus, a maximum of eight 3-amino acid sequences most frequently used for each CDR-H3 length in the mature human antibodies were designed considering their utilization frequency in the mature antibodies.

Since the contribution of CDR-H3 is larger than that of other CDRs in the antigen-antibody interaction, the larger number of CDR-H3 sequences compared to other CDRs were designed. Since 3,918 oligonucleotide sequences can be synthesized in a single array synthesis, two pools of 3,918 oligonucleotide sequences, one of which encoding CDR-H3 sequences of an even-number amino acid lengths and the other encoding CDR-H3 sequences of odd-number amino acid lengths, were designed, and thus, a total of 7,836 sequences were designed. That is, CDR-H3 sequences were designed such that sequences with 9, 11, 13, 15, 17, and 19 amino acids in length were synthesized together, while sequences with 10, 12, 14, 16, 18, and 20 amino acids in length were synthesized together.

The number of the designed CDRs as described above is presented without considering redundant sequences. With the exclusion of the repeated identical sequences, the actual numbers of unique CDR sequences were determined as shown in Table 3 below.

**[Table 3]**

| scFv library | | | | | | | | | |
|---|---|---|---|---|---|---|---|---|---|
| CDR | H1 | H2 | H3 | K1 | K2 | K3 | L1 | L2 | L3 |
| Total | 1,500 | 1,500 | 7,836 | 1,500 | 1,500 | 1,500 | 1,500 | 1,500 | 1,500 |
| Unique | 502 | 1,300 | 7,526 | 657 | 202 | 920 | 678 | 279 | 1,015 |

The concept of the design of the CDR sequences is explained in FIG. 1.
- Each of heavy chain, kappa light chain, and lambda light chain has three CDRs, in total of nine CDRs. Of these, the other CDRs excluding the first CDR of the heavy chain (CDR-H1) and the second CDRs of the kappa and lambda light chains (CDR-L2) were designed to have various lengths due to the high length diversity in the human-derived antibodies. Specifically, the CDRs were designed such that heavy chain CDR-H2 has 16 or 17 amino acids, kappa light chain CDR-L1 has 11, 12, or 16 amino acids, lambda light chain CDR-L1 has 11, 13, or 14 amino acids, kappa light chain CDR-L3 has 9 or 10 amino acids, lambda light chain CDR-L3 has 9, 10, or 11 amino acids, and heavy chain CDR-H3 has 9 to 20 amino acids. The percentage of CDR sequences having each length followed a percentage of CDR having the same length in the human-derived antibodies.

### 1-3. Excluded antibody library complementarity determining region (CDR) sequences

In the design of CDR sequences, sequences including the following post-translational modification sequences were excluded. The post-translational modification of proteins may affect the functions and physicochemical properties of the proteins, and thus it is advantageous to exclude such sequences as much as possible in the design of the antibody library.
- N-glycosylation sequences, i.e., Asp-Xaa-Ser/Thr (Asp: aspartic acid, Xaa: any of 19 kinds of amino acids other than proline, Ser/Thr: serine or threonine) were excluded.
- Isomerization sequences were excluded. It is known that aspartic acids in proteins can be spontaneously and slowly isomerized to become isoaspartic acids in an aqueous solution, while the rate of this reaction is especially affected by an amino acid residue located at the C-terminus of aspartic acid. It is known that the rate of isomerization is especially increased when the residue is glycine, and thus, CDR sequences having the aspartic acid-glycine sequence were excluded.

- Deamidation sequences were excluded. It is known that asparagines in proteins can be spontaneously and slowly deamidated to become aspartic acids in an aqueous solution, and the rate of this reaction is especially affected by an amino acid residue located at the C-terminus of asparagine. It is known that the rate of deamidation is especially increased when the residue is glycine, and thus, CDR sequences having the asparagine-glycine sequence were excluded.
- Cleavage sequences were excluded. The peptide bonds that make up proteins are slowly cleaved in an aqueous solution, and especially, such a cleavage occurs more rapidly in the aspartic acid-proline sequence, and thus, CDR sequences having the aspartic acid-proline sequence were excluded.
- Oxidation sequences were excluded. It is known that several amino acids out of 20 amino acids constituting proteins may be oxidatively modified, and especially, cysteine or methionine containing sulfur are relatively easily oxidized. Therefore, CDR sequences containing cysteine and methionine are basically excluded, while the sequences containing methionine that is already present dominantly in germline CDR sequences were not excluded. Specifically, the 34th residue of the heavy chain in CDR-H1 or the 100th residue of heavy chain in CDR-H3 correspond to this case.
- The designed CDR amino acid sequences were reverse-translated into nucleotide sequences, and the framework sequences of the antibody variable regions were added at both sides of the CDR sequence, thereby finally designing oligonucleotide sequences composed of 100 nucleotides. A total of 19,836 sequences were designed, and synthesized in the form of an oligonucleotide mixture through an array synthesis method (LC Sciences, Houston, Texas, USA).

Respective CDRs were amplified through polymerase chain reaction (PCR) from a mixture of synthesized oligonucleotides, and here, the combination of used primers was disclosed in Table 4 below. PCR conditions were as follows: 94°C, 2 min; (94°C, 30 s / 56°C, 30 s / 72°C, 30 s) repeated 25 times; 72°C, 7 min. Here, 1.6 ng of oligonucleotide mixture was used as a template for PCR reaction. Each of the primers has a concentration of 600 nM, dNTP of 0.8 mM, polymerase is Taq (2.5 units), and the reaction volume is 100 µL. After completion of the reaction, electrophoresis was carried out on 2% agarose gel, and DNA was extracted and purified from DNA bands corresponding to about 100 base pairs (bp).

**[Table 4]**

| CDR | Primer | Sequence (5' -> 3') |
|---|---|---|
| CDR-H1 | OPALS-h1-f (SEQ ID NO: 1) | CAGCGGATTCACCTTCAGC |
| CDR-H1 | OPALS-h1-b (SEQ ID NO: 2) | AGGTGCTTGGCGAACCCA |
| CDR-H2 | OPALS-h2-f (SEQ ID NO: 3) | GGCCTGGAATGGGTGAGC |
| CDR-H2 | OPALS-h2-b (SEQ ID NO: 4) | GCGGCTGATGGTAAAGCG |
| CDR-H3-odd/even | OPALS-h3-f (SEQ ID NO: 5) | GGACACCGCAGTCTACTACT |
| CDR-H3-odd/even | OPALS-h3-b (SEQ ID NO: 6) | CACCAGAGTACCTTGTCCC |
| CDR-L1 kappa | OPALS-k1-f (SEQ ID NO: 7) | CGCGCAACACTGTCATGC |
| CDR-L1 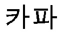 | OPALS-k1-b (SEQ ID NO: 8) | TGGAGCCTGACCTGGTTTC |
| CDR-L2 kappa | OPALS-k2-f (SEQ ID NO: 9) | CCAGGTCAGGCTCCACGT |
| CDR-L2 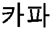 | OPALS-k2-b (SEQ ID NO: 10) | ACCGCTTCCAGATCCTGAG |
| CDR-L3 kappa | OPALS-k3-f (SEQ ID NO: 11) | CTGGAACCTGAGGACTTTG |
| CDR-L3 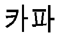 | OPALS-k3-b (SEQ ID NO: 12) | ACTTTAGTGCCCTGACCG |
| CDR-L1 lambda | OPALS-11-f (SEQ ID NO: 13) | GCGCGTGACTATTAGCTGT |
| CDR-L1 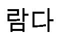 | OPALS-11-b (SEQ ID NO: 14) | AGGTGCAGTTCCAGGCAGT |
| CDR-L2 lambda | OPALS-12-f (SEQ ID NO: 15) | GCCTGGAACTGCACCTAAG |
| CDR-L2 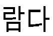 | OPALS-12-b (SEQ ID NO: 16) | GCCTGATTTGCTACCGCTA |
| CDR-L3 lambda | OPALS-13-f (SEQ ID NO: 17) | CTTCGCTCCGAAGATGAAG |
| CDR-L3 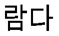 | OPALS-13-b (SEQ ID NO: 18) | GTCAGCTTGGTACCGCCA |

Codon-optimized scFv genes were synthesized (Genscript, Piscataway, NJ) for use as frameworks for antibody library production. These genes are scFv genes in which germline genes VH3-23 and VK3-A27, or VH3-23 and VL1g were connected to each other via a linker of (Gly-Gly-Gly-Gly-Ser)₃, and were cloned to pUC57 vector, and for cloning into the phagemid vector, two SfiI restriction enzyme sites, which are compatible with pComb3X vector, were included. For convenience of library construction, these genes cloned into pUC57 vector were also cloned into pComb3X vector, and then used as a template for PCR. These genes are intended to be used as frameworks, and thus are single sequences without sequence diversity, and the codons were optimized to improve the expression in mammalian cells, but in order to prevent non-specific annealing in the PCR process, the codon-optimized DNA sequences were partially changed without changing the translated amino acid sequences. The sequences of these (scFv VH3-23_linker_VK3-A27, and scFv VH3-23_linker_VL1g) were disclosed as SEQ ID NO: 19 and SEQ ID NO: 20, respectively.

### 1-4. CDR library construction

Various fragments of the codon-optimized scFv frameworks were amplified by PCR, and attached to PCR-amplified CDR DNA through overlap extension PCR, thereby constructing a single CDR library having diversity only in one CDR.

PCR conditions were the same as above, except that the elongation time at 72°C was adjusted to 30 s, 1 min, or 1 min 30 s depending on the length of predicted DNA fragment products. Specifically, the elongation time was 30 s for a predicted length of 500 base pairs or smaller, 1 min for 500 to 1,000 base pairs, and 1 min 30 s for 1,000 to 1,500 base pairs. These processes and primer sequences used herein were summarized in Tables 5 and 6 below, respectively.

**[Table 5]**

| Product name | DNA template | Primer | |
|---|---|---|---|
| Framework region PCR for constructing single CDR library | | | |
| 1 | VH3-23/VL1g-pUC57 | pUC57-b | OPALS-H1-rc-b |
| 2 | VH3-23/VL1g-pUC57 | pUC57-b | OPALS-H2-rc-b |
| 3 | VH3-23/VL1g-pUC57 | pUC57-b | OPALS-H3-rc-b |
| 4 | VH3-23/VL1g-pUC57 | pUC57-b | OPALS-L1-rc-b |
| 5 | VH3-23/VL1g-pUC57 | pUC57-b | OPALS-L2-rc-b |
| 6 | VH3-23/VL1g-pUC57 | pUC57-b | OPALS-L3-rc-b |
| 7 | VH3-23/VL1g-pComb3X | OPALS-H1-rc-f | pC3X-b |
| 8 | VH3-23/VL1g-pComb3X | OPALS-H2-rc-f | pC3X-b |
| 9 | VH3-23/VL1g-pComb3X | OPALS-H3-rc-f | pC3X-b |
| 10 | VH3-23/VL1g-pComb3X | OPALS-L1-rc-f | pC3X-b |
| 11 | VH3-23/VL1g-pComb3X | OPALS-L2-rc-f | pC3X-b |
| 12 | VH3-23/VL1g-pComb3X | OPALS-L3-rc-f | pC3X-b |
| 13 | VH3-23/VK3-A27-pComb3X | OPALS-K1-rc-f | pC3X-b |
| 14 | VH3-23/VK3-A27-pComb3X | OPALS-K2-rc-f | pC3X-b |
| 15 | VH3-23/VK3-A27-pComb3X | OPALS-K3-rc-f | pC3X-b |
| 26 | VH3-23/VK3-A27-pUC57 | pUC57-b | OPALS-K1-rc-b |
| 27 | VH3-23/VK3-A27-pUC57 | pUC57-b | OPALS-K2-rc-b |
| 28 | VH3-23/VK3-A27-pUC57 | pUC57-b | OPALS-K3-rc-b |
| Single CDR library construction | | | |
| H1 | 1 + CDR-H1 + 7 | pC3x-b | pUC57-b |
| H2 | 2 + CDR-H2 + 8 | | pUC57-b |
| Odd | 3 + CDR-H3-odd + 9 | | pUC57-b |
| Even | 3 + CDR-H3-even + 9 | | pUC57-b |
| λ1 | 4 + CDR-L1-lambda + 10 | | pUC57-b |
| λ2 | 5 + CDR-L2-lambda + 11 | | pUC57-b |
| λ3 | 6 + CDR-L3-lambda + 12 | | pUC57-b |
| κ1 | 26 + CDR-L1-kappa + 13 | | pUC57-b |
| κ2 | 27 + CDR-L2-kappa + 14 | | pUC57-b |
| κ3 | 28 + CDR-L3-kappa + 15 | | pUC57-b |

**[Table 6]**

| Primer | Sequence (5'->3') |
|---|---|
| OPALS-H1-rc-f (SEQ ID NO: 21) | TGGGTTCGCCAAGCACCT |
| OPALS-H2-rc-f (SEQ ID NO: 22) | CGCTTTACCATCAGCCGC |
| OPALS-H3-rc-f (SEQ ID NO: 23) | GGGACAAGGTACTCTGGTG |
| OPALS-L1-rc-f (SEQ ID NO: 24) | ACTGCCTGGAACTGCACCT |
| OPALS-L2-rc-f (SEQ ID NO: 25) | TAGCGGTAGCAAATCAGGC |
| OPALS-L3-rc-f (SEQ ID NO: 26) | TGGCGGTACCAAGCTGAC |
| OPALS-K1-rc-f (SEQ ID NO: 27) | GAAACCAGGTCAGGCTCCA |
| OPALS-K2-rc-f (SEQ ID NO: 28) | CTCAGGATCTGGAAGCGGT |
| OPALS-K3-rc-f (SEQ ID NO: 29) | CGGTCAGGGCACTAAAGT |
| OPALS-H1-rc-b (SEQ ID NO: 30) | GCTGAAGGTGAATCCGCTG |
| OPALS-H2-rc-b (SEQ ID NO: 31) | GCTCACCCATTCCAGGCC |
| OPALS-H3-rc-b (SEQ ID NO: 32) | AGTAGTAGACTGCGGTGTCC |
| OPALS-L1-rc-b (SEQ ID NO: 33) | ACAGCTAATAGTCACGCGC |
| OPALS-L2-rc-b (SEQ ID NO: 34) | CTTAGGTGCAGTTCCAGGC |
| OPALS-L3-rc-b (SEQ ID NO: 35) | CTTCATCTTCGGAGCGAAG |
| OPALS-K1-rc-b (SEQ ID NO: 36) | GCATGACAGTGTTGCGCG |
| OPALS-K2-rc-b (SEQ ID NO: 37) | ACGTGGAGCCTGACCTGG |
| OPALS-K3-rc-b (SEQ ID NO: 38) | CAAAGTCCTCAGGTTCCAG |
| pC3x-b (SEQ ID NO: 39) | AACCATCGATAGCAGCACCG |
| pUC57-b (SEQ ID NO: 40) | TTCGCCATTCAGGCTGCG |

The constructed single CDR library genes were isolated and purified through 1% agarose gel electrophoresis, and cleaved with SfiI restriction enzyme at 50°C for 12 hours or longer, and then ligated to the pComb3X phagemid vector, which has been cleaved with SfiI restriction enzyme in the same manner. 1 µg of vector and 1 µg of scFv DNA were reacted at room temperature for 16 hours in 50 µl T4 ligase buffer containing 1,000 units of T4 ligase. Then, 5 µl of 3 M sodium acetate (pH 5.2) and 110 µl of ethanol were added to the reaction product to precipitate DNA at -20°C for 1 hour or longer, and the precipitated DNA was centrifuged, washed with cold 70% ethanol, and dissolved in 20 µl of sterilized pure water. This product was transformed to *E*. *coli* ER2537 by electroporation, and cultured in media containing ampicillin antibiotic for 12-16 hours, thereby obtaining single CDR libraries.

The purpose of constructing a single CDR library is to remove CDR sequences that cause inaccuracy and premature translational termination due to insertion/deletion/substitution of nucleotides present in the synthesized CDR oligonucleotide. *E. coli* transformed with the single CDR libraries was incubated, and in the log phase, helper phages and kanamycin were added successively, followed by incubation overnight, to obtain phage libraries. These single-CDR phage libraries were subjected to one round of panning against anti-HA antibody adsorbed on a surface of the immunotube, to select only phage clones expressing HA-tag. The HA-tag is a short peptide sequence composed of nine amino acids (YPYDVPDYA) and is located at the 3 'end of the SfiI cloning site of pComb3X phagemid vector, so that the HA-tag is present at the C-terminus of the expressed scFv protein. Since the efficiency of chemical synthesis of oligonucleotides is not perfect, there are many sequences having insertion/deletion/substitution causing premature translational termination in the synthesized CDR oligonucleotides, and phage clones containing the sequences may not have HA-tag, and thus be removed by panning. Resultantly, non-defective CDR sequences were selected through the above processes.

### 1-5. scFv antibody library construction

The selected CDR sequences were amplified through PCR according to the above-described conditions, and the amplified DNA fragments were again combined into heavy chain and light chain variable regions through overlap extension PCR. A linker was ligated to the light chain variable region by overlap extension PCR, and the resulting product was again combined with the heavy chain variable region by overlap extension PCR, to finally obtain an scFv gene library. A PCR procedure for constructing the final library was depicted in FIG. 2. These processes and primer sequences used herein were summarized in Tables 7 and 8 below.

**[Table 7]**

| Product name | Template | Primer | |
|---|---|---|---|
| Amplification of selected CDR and linker | | | |
| VH-CDR1 | H1 | pC3x-f | OPALS-H2-rc-b |
| VH-CDR2 | H2 | OPALS-H1-rc-f | OPALS-H3-rc-b |
| VH-CDR3-odd | Odd | OPALS-H2-rc-f | OPALS-FR4-b |
| VH-CDR3-even | Even | OPALS-H2-rc-f | OPALS-FR4-b |
| VL-CDRλ1 | λ1 | OPALS-L1-f | OPALS-L2-rc-b |
| VL-CDRλ2 | λ2 | OPALS-L1-rc-f | lFR3-b |
| VL-CDRλ3 | λ3 | lFR3-f | pC3x-b |
| Linker-λ | OPALS-lambda | OPALS-FR4-f | OPALS-L1-rc-b |
| VL-CDRκ1 | κ1 | kFR1-f | OPALS-K2-rc-b |
| VL-CDRκ2 | κ2 | OPALS-K1-rc-f | OPALS-K3-rc-b |
| VL-CDRκ3 | κ3 | OPALS-K2-rc-f | pC3x-b |
| Linker-κ | OPALS-kappa | OPALS-FR4-f | kFR1-b |
| Amplification of heavy chain and light chain variale regions | | | |
| VH-Odd | VH-(CDR1+CDR2+CDR3-odd) | pC3x-f | OPALS-FR4-b |
| VH-Even | VH-(CDR1+CDR2+CDR3-even) | pC3x-f | OPALS-FR4-b |
| VL-Lambda | VL-(CDRλ1+λ2+λ3) | OPALS-L1-f | pC3x-b |
| VL-Kappa | VL-(CDRκ1+κ2+κ3) | OPALS-K1-f | |
| Linkage beweeen light chain variable region and linker | | | |
| λ | VL-Lambda+linker-λ | OPALS-FR4-f | pC3x-b |
| K | VL-Kappa+linker-κ | OPALS-FR4-f | pC3x-b |
| Final PCR for constructing ScFv library | | | |
| Oλ | VH-Odd+λ | pC3-seq | dp-seq |
| Eλ | VH-Even+λ | pC3-seq | dp-seq |
| Oκ | VH-Odd+κ | pC3-seq | dp-seq |
| Eκ | VH-Even+κ | pC3-seq | dp-seq |

**[Table 8]**

| Primer | Sequence (5'→3') |
|---|---|
| pC3x-f (SEQ ID NO: 41) | GCACGACAGGTTTCCCGAC |
| lFR3-f (SEQ ID NO: 42) | CTGGCCATCAGCGGCCTTC |
| lFR3-b (SEQ ID NO: 43) | CTGGGTCAGCACGATTTC |
| kFR1-f (SEQ ID NO: 44) | GAAATCGTGCTGACCCAG |
| kFR1-b (SEQ ID NO: 45) | CTGGGTCAGCACGATTTC |
| OPALS-FR4-f (SEQ ID NO: 46) | CTGGTGACCGTGAGCAGC |
| OPALS-FR4-b (SEQ ID NO: 47) | GCTGCTCACGGTCACCAG |
| pC3-seq (SEQ ID NO: 48) | GTGAGCGGATAACAATTGA |
| dp-seq (SEQ ID NO: 49) | AGAAGCGTAGTCCGGAACG |

The scFv library was completed by the above-described method such that the scFv gene library was cleaved with SfiI restriction enzyme, ligated to pComb3X vector, and then transformed to *E. coli* ER2537. Specifically, libraries having even and odd numbers of amino acids (length) in CDR-H3, and libraries having kappa and lambda light chains, were separately constructed, and these were subjected to PCR combination and ligation-transformation, so that a total of four sub-libraries were constructed. These sub-libraries were called OL (odd-lambda), EL (even-lambda), OK (odd-kappa) and EK (even-kappa) libraries, and the titers of bacteria transformed with the respective libraries, that is, the sizes of the libraries were 1.3x10⁸, 1.4x10⁸, 1.9x10⁸, and 3.7x10⁸, respectively, and thus, a primary library having a total size of 8.3x10⁸ was constructed. A secondary library having a total size of 10¹⁰ were later prepared following the same experimental procedure. The sequence comparison between the constructed OL library and EL library is shown in FIG. 5a, while the sequence comparison between the OK library and the EK library is shown in FIG. 5b.

After 24 clones were arbitrarily selected from each sub-library, the bacteria were incubated, and the expression of scFv antibody was induced by IPTG, followed by incubation at 30°C overnight. A periplasmic extract was obtained therefrom by osmotic shock using a sucrose buffer solution, and then 1 µl of periplasmic extract was blotted to nitrocellulose membrane. After drying, the membrane surface was blocked by the immersion in a 3% non-fat dried milk buffer solution, and allowed to bind to anti-HA antibody-horseradish peroxidase (HRP) for 1 hour, followed by washing. The expression of scFv was confirmed by detecting the presence of HA-tag using luminol (FIG. 3).

The construction steps for the antibody library in Example 1 were summarized in FIG. 4.

### Example 2: Sequence analysis

Next generation sequencing (NGS) of the library was performed to assess the accuracy with which the CDR design was reflected in the constructed library. Millions of CDR sequences were analyzed and compared with the designed sequences (Table 9).

**[Table 9]**

| Next-generation sequencing analysis of the library CDRs | | | | | | | | | |
|---|---|---|---|---|---|---|---|---|---|
| CDR | H1 | H2 | H3 | K1 | K2 | K3 | L1 | L2 | L3 |
| Processed sequences (x 10⁶) | 4.75 | 4.70 | 4.53 | 2.58 | 2.31 | 2.52 | 2.14 | 2.12 | 2.09 |
| In-frame % | 91.3 | 89.7 | 90.3 | 92.2 | 91.6 | 93.2 | 91.5 | 78.7 | 90.7 |
| Designed length % | 89.4 | 88.4 | 85.6 | 87.1 | 82.3 | 90.7 | 84.6 | 56.2 | 89.4 |
| Designed sequence % | 80.9 | 52.0 | 51.8 | 62.4 | 70.3 | 74.3 | 56.2 | 47.6 | 67.2 |
| In-fame % before proofreading | 85.7 | 50.0 | 67.9 | 53.3 | 44.4 | 66.7 | 38.5 | 31.3 | 66.7 |
| Design coverage % | 100 | 100 | 97.3 | 99.8 | 100 | 99.9 | 100 | 100 | 100 |

Although, for long CDRs, a majority of the unique sequences occur only once or twice in the designed repertoire and the coefficients of determination (r²) are relatively low, the designed sequences were nearly completely covered by the constructed library, and the frequency of occurrence of each designed CDR sequence also was roughly represented in the actual library (FIG. 6). Not surprisingly, the corresponding library was found to contain many low-frequency CDR sequences which were not matching any of the designed sequences due to synthesis errors, including non-functional sequences with nucleotide insertions or deletions that cause frameshift. The non-functional CDR sequences were removed through the proofreading panning of the single-CDR libraries using anti-HA antibody, and the percentage of functional in-frame CDR sequences was about 90-93% after the proofreading panning compared with 31-86% before the proofreading. The in-frame percentage of CDR-L2 (lambda) was only 79%, which was likely due to inaccurate annealing during the overlap extension PCR. The percentage of CDR sequences with the designed lengths was about 82-91% (56% for lambda CDR-L2).

Overall, about 75 % of V_{H}, V_{κ}, and V_{λ} sequences were functional variable domains without stop codons, and the percentage of functional scFv clones in the library was estimated to be approximately 55%. This estimation was roughly comparable with the dot-blot assay of randomly chosen library clones. For dot-blot assay, a periplasmic extract of randomly chosen scFv clones from the unselected library was blotted on a nitrocellulose membrane, and the presence of solubly expressed scFv in the extract was probed by detecting the C-terminus HA tag. As a result, it was estimated that approximately 60% of the clones were solubly expressed scFv (FIG. 3).

Unique variable domain sequences out of total sequenced variable domains were analyzed from NGS data. Approximately 1.3 x 10⁶ of each of the heavy, kappa, and lambda variable domain sequences without stop codons were analyzed, while 98% of V_{H}, 89% of V_{κ}, and 98% of V_{λ} sequences were non-redundant (FIG. 7).

The percentages of the number of different variable domain sequences among total sequence reads were 99%, 92%, and 99% for V_{H}, V_{κ}, and V_{λ}, respectively. Meanwhile, these values were comparable with the CDR-H3 sequence uniqueness of 97-98% for other highly diverse antibody libraries, and suggest that the redundancy among scFv clones in the unselected library is not significant.

The distribution of CDR length, especially of CDR-H3 length, in the constructed library differed from the design, with shorter length CDRs conspicuously overrepresented when compared with longer CDRs (FIG. 8). This is probably in part because of the inaccuracy during the oligonucleotide array synthesis that introduced frameshift and premature stop codons. Because these errors are more likely to occur during the synthesis of longer CDRs and most of them would be removed during the proofreading panning of the single-CDR libraries using anti-HA-tag antibody, it is considered that more of the longer CDRs were removed from the library. Also, it is possible that scFvs with shorter CDRs were preferentially selected and amplified by the panning against anti-HA-tag antibody.

The similarity of the library CDR sequences to the natural CDR sequences was assessed by analyzing the number of amino acid differences in each CDR sequence from the closest germline CDR sequence. Because the CDRs were designed to simulate the natural somatic hypermutation (SHM) patterns, it was assumed that the designed sequences are highly nature-like. Therefore, the average numbers of mutations per CDR sequence were compared between the designed and the natural CDR sequences (Table 10).

**[Table 10]**

| Average numbers of amino acid differences in the CDR sequences from the closest human germline CDR sequences | | | | | | | | | | | | | | | | |
|---|---|---|---|---|---|---|---|---|---|---|---|---|---|---|---|---|
| CDR | H1 | H2 | | K1 | | | K2 | K3 | | L1 | | | L2 | L3 | | |
| Length | 5 | 16 | 17 | 11 | 12 | 16 | 7 | 9 | 10 | 11 | 13 | 14 | 7 | 9 | 10 | 11 |
| Designed | 0.75 | 2.14 | 2.55 | 1.0 | 1.18 | 1.85 | 0.47 | 0.73 | 0.65 | 1.03 | 1.10 | 0.98 | 0.61 | 0.83 | 1.18 | 0.70 |
| Non-designed | 1.84 | 4.30 | 3.67 | 2.77 | 3.44 | 3.82 | 2.01 | 1.55 | 1.45 | 2.82 | 3.22 | 2.75 | 1.85 | 1.67 | 2.11 | 1.45 |
| Natural | 0.82 | 2.09 | 2.37 | 1.08 | 1.29 | 0.93 | 0.49 | 0.72 | 0.54 | 0.95 | 1.10 | 1.00 | 0.66 | 0.88 | 1.14 | 0.75 |

When the library CDR sequences (non-designed CDR sequences) that did not match any of the designed sequences due to synthesis errors were analyzed, the average number of amino acid differences from the closest germline CDR sequence was different from that of the designed sequences by only 1-2 amino acids on average. These results suggest that the CDR sequences of the library contain only small numbers of mutations from the human germline CDR sequences, and are highly similar to the CDR sequences of natural human antibodies. For CDR-H3, the amino acid distribution at each position was analyzed (FIG. 9). Highly similar distribution patterns were found among the CDR-H3s of natural human antibodies, the simulated repertoire, and the constructed library, demonstrating the nature-likeness of the library CDRs.

As expected, the frequency of occurrence of undesirable post-translation modification (PTM) motifs in the CDRs was much lower than that in natural human antibody CDRs, with the exceptions of CDR-H1 and CDR-L2, which are short (5 and 7 amino acids, respectively) and have relatively few PTM motifs in natural human antibodies (Table 11).

**[Table 11]**

| Percentages of post-translational modification motifs in the CDRs of the library and the natural human antibodies | | | | | | | | | | |
|---|---|---|---|---|---|---|---|---|---|---|
| Motif | CDR | H1 | H2 | H3 | K1 | K2 | K3 | L1 | L2 | L3 |
| Asp-Gly | Library | 0.05 | 0.31 | 0.66 | 0.03 | 0.34 | 0.10 | 0.32 | 3.41 | 0.34 |
| Asp-Gly | Natural | 0.08 | 10.79 | 6.45 | 6.03 | 0.32 | 0.09 | 0.28 | 0.30 | 1.98 |
| Asn-Gly | Library | 0.04 | 0.62 | 0.32 | 0.05 | 0.63 | 0.12 | 0.60 | 4.51 | 0.32 |
| Asn-Gly | Natural | 0.02 | 5.96 | 3.03 | 4.54 | 0.53 | 0.50 | 0.06 | 0.12 | 7.61 |
| Asp-Pro | Library | 0.01 | 0.16 | 0.25 | 0.04 | 0.08 | 0.10 | 0.04 | 0.20 | 0.05 |
| Asp-Pro | Natural | 0.00 | 2.40 | 10.13 | 0.00 | 0.04 | 0.14 | 0.11 | 0.00 | 0.66 |
| N-glyc | Library | 0.66 | 1.91 | 2.77 | 1.35 | 1.46 | 1.00 | 1.97 | 0.94 | 1.30 |
| N-glyc | Natural | 0.59 | 0.78 | 7.73 | 0.61 | 0.07 | 1.69 | 6.74 | 0.30 | 8.20 |
| Met | Library | 0.35 | 1.03 | 1.80 | 0.90 | 0.55 | 0.29 | 1.78 | 0.70 | 1.70 |
| Met | Natural | 0.06 | 2.72 | 9.04 | 0.88 | 0.11 | 12.36 | 0.39 | 0.18 | 0.86 |
| Cys | Library | 0.30 | 1.96 | 0.49 | 1.30 | 0.64 | 0.77 | 2.10 | 0.87 | 1.29 |
| Cys | Natural | 0.35 | 17.50 | 1.46 | 0.92 | 0.18 | 1.19 | 0.61 | 0.24 | 1.98 |
| Total PTM | Library | 1.36 | 5.44 | 5.87 | 3.60 | 3.58 | 2.32 | 6.16 | 10.48 | 4.81 |
| 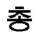 PTM | Natural | 1.09 | 36.70 | 32.92 | 12.55 | 1.23 | 15.56 | 8.08 | 1.13 | 20.70 |

Assuming that the PTM motifs occur independently in different CDRs, the probability of at least one PTM motifs occurring in an scFv sequence in the library was estimated to be approximately 20-30% (70-80% of the clones without PTM motifs), whereas only between 24 to 27% of scFvs from natural sources would be free of the PTM motifs.

### Example 3: Panning and screening of the library against antigens

The constructed library was panned against four antigens to validate its functionality. Multiple target-binding scFv clones were isolated from the library after four rounds of panning against antigens fixed on the plastic surface. Output colonies from the third or fourth round of panning were screened by ELISA, and some of the clones with positive signal were sequenced (Table 12).

**[Table 12]**

| Panning and screening of the library against antigens | | | | |
|---|---|---|---|---|
| Antigen | Number of panning | ELISA positive/screened | Unique sequences/ Total sequenced | Kappa/lambda |
| AIMP1 | 3 | 10/94 | 6/6 | 1/5 |
| SerRS | 4 | 16/94 | 3/14 | 0/14 |
| hEpCAM-ECD | 4 | 18/94 | 3/15 | 3/12 |
| HER3-ECD | 4 | 46/188 | 6/16 | 8/8 |
| HEW Lysozyme | 4 | 151/188 | 6/13 | 1/12 |

Although the number of the antigens tested and the clones sequenced were not enough for the generalization of the light chain class preference, a majority of the isolated clones were of lambda light chain class. The preferential selection of clones with specific light chain family/class from phage antibody libraries has been reported previously, and has been attributed to the preferential pairing of specific VH-VL domains and the difference in the functional sizes of the sub-libraries. Strong preference for lambda light chain after antigen-driven phage display selection of large natural scFv libraries has also been reported, suggesting that the lambda chain preference may be a more-or-less universal phenomenon of the phage display selection of scFv libraries, rather than a characteristic of a specific antibody library.

Binding kinetics of some of the ELISA-positive scFv clones isolated from the library were analyzed by surface plasmon resonance (SPR) (Table 13).

**[Table 13]**

| Binding kinetics of selected target-specific scFv clones determined by SPR | | | |
|---|---|---|---|
| Antigen-clone | Kₒₙ (M⁻¹s⁻¹) | K_{off} (s⁻¹) | K_{D} (M) |
| HER3-G3 | 6.8 x 10⁵ | 2.8 x 10⁻³ | 4.2 x 10⁻⁹ |
| HER3-C5 | 1.2 x 10⁵ | 1.0 x 10⁻³ | 8.1 x 10⁻⁹ |
| HER3-D11 | 7.8 x 10⁴ | 5.3 x 10⁻³ | 6.8 x 10⁻⁸ |
| HEWL-B6 | 1.1 x 10⁵ | 5.3 x 10⁻³ | 4.8 x 10⁻⁸ |
| HEWL-F8 | 5.8 x 10⁴ | 1.9 x 10⁻³ | 3.3 x 10⁻⁸ |
| HEWL-B2 | 1.1 x 10⁶ | 3.0 x 10⁻³ | 2.8 x 10⁻⁹ |
| HEWL-G7 | 1.4 x 10⁵ | 3.2 x 10⁻³ | 2.4 x 10⁻⁸ |
| SRS-A3 | 2.7 x 10⁴ | 1.8 x 10⁻³ | 6.7 x 10⁻⁸ |
| SRS-C4 | 9.5 x 10⁴ | 1.3 x 10⁻³ | 1.4 x 10⁻⁸ |
| SRS-D8 | 6.5 x 10⁴ | 1.4 x 10⁻³ | 2.2 x 10⁻⁸ |

Dissociation constants (Kd) ranging from 10⁻⁹ to 10⁻⁷ M were obtained for 10 scFv fragments against three different antigens.

The NGS results from the unselected library were compared with the sequences of the unique scFv fragments selected from the library after panning. The panning process did not appear to significantly alter the percentage of the designed CDR sequences or the average number of mutations per CDR residue (Table 14)

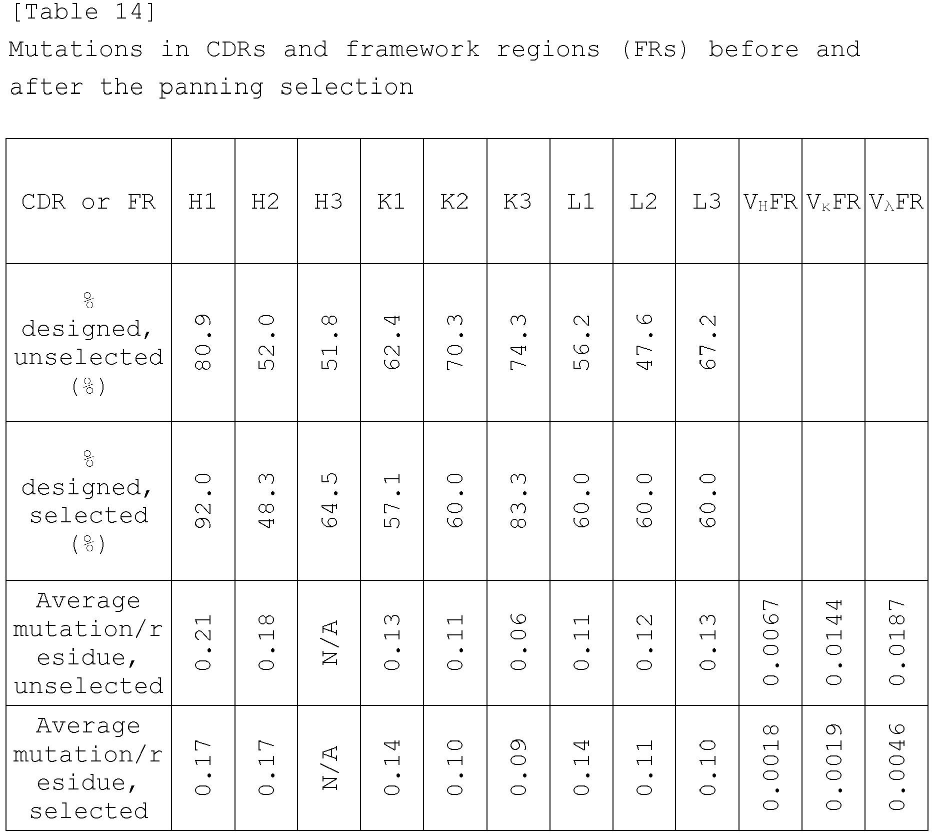

It can be confirmed that the additional CDR diversity introduced by the errors in oligonucleotide synthesis or PCR was not critical to the performance of the library. On the other hand, it was confirmed that the mutation frequency in framework regions (FRs) decreased after the panning selection.

### Example 4: Specific validation of library construction

In order to validate the construction of antibody libraries through Examples 1-3, scFv sequences were analyzed. Any clones were selected from from each sub-library and scFv genes were amplified through PCR technique, and then DNA was purified and subjected to sequencing. The sequencing analysis results are shown in FIG. 5, indicating that CDRs having various designed sequences and lengths were introduced into scFv clones constituting the library.

As a result of specific sequencing analysis, it was confirmed that there were no unintended post-translational modification sequences except for one case in which the cleavage (aspartic acid-proline) sequence was introduced by a PCR error out of 18 scFv sequences. Whereas, from a result of analyzing the percentage of the occurrence of sequences that could cause such post-translational modifications in each CDR of human-derived mature antibodies, the percentage was 5.7% for CDR-H1, 39.7% for CDR-H2, and 34.5% for CDR-H3 in the heavy chain; 12.6% for CDR-L1, 0.6% for CDR-L2, and 15.9% for CDR-L3 in the kappa light chain; and 8.3% for CDR-L1, 6.4% for CDR-L2, and 24.0% for CDR-L3 in the lambda light chain. Therefore, it was confirmed that the antibody library capable of minimizing post-translational modification as designed was constructed.

The transformed *E. coli* library was incubated, and infected with VCSM13 helper phage to obtain an scFv-displayed antibody phage library (H. Y. Yang et al., Mol. Cells 2009, 27, 225-235). This library contains more than 10¹² colony forming units (CFU) per ml. Using this antibody, the functionality of the antibody phage library was validated by a panning experiment of selecting antigen-specific antibodies against lysozyme (Hen egg white lysozyme, HEWL) and AIMP1 (101-192) antigens. Thereafter, 10 ug/ml of an antigen solution was added to an immunotube to allow a protein to be adsorbed onto a surface of the tube for 1 hour, and then a 3% nonfat dried milk buffer solution was added to the tube to protect the surface to which antigens were not adsorbed.

After the tube was emptied, 10¹² CFU of antibody phage libraries dispersed in a 3% nonfat dried milk buffer solution were added thereto, and then allowed to bind to antigens for 1-2 hours. Non-specifically bound phages were washed out three times with TBST (tris buffered saline - tween 20) solution, and then the remaining antigen-specific phage antibodies were eluted using 100 mM triethylamine solution.

The eluted phages were neutralized with 1.0 M Tris-HCl buffer (pH 7.8), and then added to *E. coli* ER2537 at 37 °C for 1 hour, and the infected *E. coli* cells were plated on ampicillin & 2% glucose-containing LB (Luria-Bertani) agar medium, followed by incubation at 37 °C.

On the next day, the incubated E. coli cells were suspended in 5 mL of super broth (SB) medium, and 15% glycerol was added thereto. The suspension was stored at -80 °C, and 50 µl thereof was added 20 ml of SB-ampicillin solution, followed by culture at 37°C.

When the absorbance of the culture liquid at 600 nm reached 0.5, 10¹¹ PFU (plaque forming unit) of VCSM13 helper phages were added, followed by incubation at 37°C with slow shaking. After 1 hour, 70 µg/ml kanamycin was added, followed by vigorous shacking (220 rpm) at 30°C overnight. Next day, the culture was centrifuged, and then 5 ml of 5x PEG precipitation solution (20% w/v PEG8000, 15% w/v NaCl) was added to the supernatant, followed mixing. The mixture was left on ice for 30 minutes or longer to precipitate phages, and the precipitated phages were centrifuged and dispersed in 300 µl of PBS. The phage solution was again centrifuged to take only the supernatant, and 700 µl of a 3% nonfat dried milk buffer solution was added thereto, and panning was performed as above using the phage solution to enrich antigen-specific clones. After three or four runs of panning, *E. coli* containing antibody genes were plated on LB agar medium containing ampicillin and 2% glucose, followed by incubation, thereby obtaining single colonies, which were then inoculated and incubated in 200 µl of SB-ampicillin solution, and the expression of scFv protein in the periplasm of *E. coli* was induced by IPTG.

Periplasmic extracts were obtained from *E. coli* using osmotic shock using a sucrose buffer solution, and then, the periplasmic extracts were used to examine the binding between the antigen and scFv using ELISA technique (H. Y. Yang et al., Mol. Cells 2009, 27, 225-235). The bound scFvs were detected using anti-HA antibody-HRP and tetramethylbenzidine (TMB). Antigen-specific antibody clones identified therefrom were analyzed through sequencing analysis.

188 clones were screened by ELISA against HEWL antigen, and 150 ELISA positive clones showing a binding signal by 3-fold or higher compared with the background signal were identified, of which 16 clones were sequenced to find five unique sequences. 94 clones for AIMP1 (101-192) were screened by ELISA, and 18 ELISA positive clones showing a binding signal by 3-fold or more compared with a background signal were identified, of which five clones were sequenced to find two unique sequences.

In summary, the antibody library constructed by the present invention contains antibodies having excellent physicochemical properties against a plurality of antigens, thereby confirming that it can be favorably used as an antibody library having functional diversity and including a plurality of unique sequences.

While the present invention has been described with reference to the particular illustrative embodiments, those skilled in the art to which the present invention pertains can understand that the present invention may be embodied in other specific forms without departing from the technical spirit or essential characteristics thereof. Therefore, the embodiments described above should be construed as exemplary, not limiting the present invention in any manner. The scope of the present invention is not defined by the detailed description as set forth above but by the accompanying claims of the invention, and it should also be understood that all changes or modifications derived from the definitions and scopes of the claims and their equivalents fall within the scope of the invention.

## Claims

1. A method for preparing an antibody library, the method comprising:
individually designing complementarity determining region (CDR) sequences of antibodies; and
synthesizing antibodies comprising the designed complementarity determining region sequences to prepare a library.

2. The method of claim 1, wherein heavy chain complementarity determining region 1 (CDR-H1), heavy chain complementarity determining region 2 (CDR-H2), heavy chain complementarity determining region 3 (CDR-H3), light chain complementarity determining region 1 (CDR-L1), light chain complementarity determining region 2 (CDR-L2), and light chain complementarity determining region 3 (CDR-L3), which constitute the complementarity determining regions of the antibodies included in the antibody library, have diversity.

3. The method of claim 1, wherein in the individual designing of the complementarity determining region sequences, for CDR-H1, CDR-H2, CDR-L1, or CDR-L2, the sequences therefor are designed by simulating i) an utilization frequency of each germline immunoglobulin gene, ii) a frequency of mutation into each of 20 amino acids by somatic hypermutations at each amino acid, iii) a length distribution frequency of sequences comprising each complementarity determining region, or iv) a frequency of each amino acid at each position calculated by analyzing a combination thereof, of the complementarity determining regions of actual human-derived mature antibodies.

4. The method of claim 1, wherein in the individual designing of the complementarity determining region sequences, for CDR-L3,
a) 7 or 8 amino acid sequences from a N-terminus of the complementarity determining region are designed by simulating i) an utilization frequency of each germline immunoglobulin gene, ii) a frequency of mutation into each of 20 amino acids by somatic hypermutations at each amino acid position, iii) a length distribution frequency of sequences comprising CDR-L3, or iv) a frequency of each amino acid at each position calculated by analyzing a combination thereof, of the complementarity determining region of actual human-derived mature antibodies, and
b) 2 or 3 amino acid sequences from a C-terminus of the complementarity determining region are designed by analyzing and calculating a frequency of each amino acid at each position in the complementarity determining region of actual human-derived mature antibodies, then simulating sequences that reflect the calculated frequencies; and
wherein the CDR-L3 contains 9 to 11 amino acids and the analysis of the frequencies is conducted according to each length, the CDR-L3 sequences being designed based on an analysis result of complementarity determining region CDR-L3 of human-derived mature antibodies, which have the same amino acid lengths as CDR-L3 to be designed.

5. The method of claim 1, wherein, when a light chain complementarity determining region sequence is designed, the light chain is a kappa light chain or a lambda light chain.

6. The method of claim 1, wherein in the individual designing of the complementarity determining region sequences, for CDR-H3,
a) each sequence therefor excluding three amino acids from a C-terminus of the complementarity determining region is designed by using a frequency of each amino acid at each position in the complementarity determining region of actual human-derived mature antibodies, and
b) a 3 amino acid sequence from the C-terminus of the complementarity determining region is designed by analyzing and calculating frequencies of the corresponding 3 amino acid sequences in the complementarity determining region of actual human-derived mature antibodies, then simulating sequences that reflect the calculated frequencies; and
wherein the CDR-H3 contains 9 to 20 amino acids and the analysis of the frequencies is conducted according to each length, the CDR-H3 sequences being designed based on an analysis result of complementarity determining region CDR-H3 of human-derived mature antibodies, which have the same amino acid length as CDR-H3 to be designed.

7. The method of claim 1, further comprising, after the designing of the complementarity determining region amino acid sequences,
excluding sequences having N-glycosylation, isomerization, deamidation, cleavage, and oxidation motifs from the designed sequences.

8. The method of claim 1, further comprising, after the designing of the complementarity determining region amino acid sequences,
reverse-translating the designed sequences into polynucleotide sequences and then designing oligonucleotide sequence in which framework region sequences of variable regions of a human antibody germline gene flanking the complementarity determining region are linked to the 5' and 3' ends of the reverse-translated polynucleotide.

9. The method of claim 1, wherein the antibodies include amino acid sequences encoded by VH3-23 (Genebank accession No. Z12347), VK3-A27 (Genebank accession No. X93639), VL1g (GenBank accession No. Z73663), or fragments thereof.

10. The method of claim 1, wherein the antibodies are selected from the group consisting of IgA, IgD, IgE, IgM, IgG, Fc fragments, Fab, Fab', F(ab')₂, scFv, single variable domain antibody, and Fv.

11. The method of claim 3, wherein the method corresponds to at least one of 1) to 6) below:
1) using an amino acid sequence of SEQ ID NO: 54 to SEQ ID NO: 84 as the germline CDR sequence for the heavy chain CDR-H1;
2) using an amino acid sequence of SEQ ID NO: 85 to SEQ ID NO: 121 as the germline CDR sequence for the heavy chain CDR-H2;
3) using a kappa light chain for the germline CDR sequence used for the designing of the light chain CDR and using an amino acid sequence of SEQ ID NO: 122 to 145 as the germline CDR sequence for kappa light chain CDR-L1;
4) using a kappa light chain for the germline CDR sequence used for the designing of the light chain CDR and using an amino acid sequence of SEQ ID NO: 146 to 165 as the germline CDR sequence for kappa light chain CDR-L2;
5) using a lambda light chain for the germline CDR sequence used for the designing of the light chain CDR and using an amino acid sequence of SEQ ID NO: 166 to 189 as the germline CDR sequence for lambda light chain CDR-L1; and
6) using a lambda light chain for the germline CDR sequence used for the designing of the light chain CDR and using an amino acid sequence of SEQ ID NO: 190 to 209 as the germline CDR sequence for lambda light chain CDR-L2.

12. The method of claim 4, wherein the method corresponds to at least one of 1) to 2) below:
1) using a kappa light chain for the germline CDR sequence used for the designing of the light chain CDR and using an amino acid sequence of SEQ ID NO: 210 to 236 as the germline CDR sequence for kappa light chain CDR-L3; and
2) using a lambda light chain for the germline CDR sequence used for the designing of the light chain CDR and using an amino acid sequence of SEQ ID NO: 237 to 252 as the germline CDR sequence for lambda light chain CDR-L3.

13. The method of claim 11, wherein an utilization frequency of each germline CDR sequence simulates the utilization frequency of each germline CDR sequence in natural human antibodies, which is obtained through the analysis of antibody sequence databases.

14. The method of claim 12, wherein an utilization frequency of each germline CDR sequence simulates the utilization frequency of each germline CDR sequence in natural human antibodies, which is obtained through the analysis of antibody sequence databases.

15. An antibody library prepared by the method of any one of claims 1 to 14.
